# EUROPEAN PATENT APPLICATION

(11) **EP 2 226 396 A1**
(43) Date of publication of application: **08.09.2010**
(21) Application number: 10158352.4
(22) Date of filing: 28.05.2004
(51) Int. Cl.: C12Q 1/68

(54) **Gene expression markers for response to EGFR inhibitor drugs**

(30) Priority: 30.05.2003 US 474908 P
(62) Divisional of application: 04753936.6
(71) Applicant: Genomic Health, Inc., Redwood City, CA 94063 (US); Cedars-Sinai Medical Center, Los Angeles, CA 90048 (US)
(72) Inventor: Baker, Joffre B., Montara, CA 94937 (US); Shak, Steve, Burlingame, CA 94010 (US); Agus, David, Beverly Hills, CA 90210 (US); Natale, Ronald, Santa Monica, CA 90402 (US)
(74) Representative: Tollett, Ian

(57) **Abstract**

The present invention concerns prognostic markers associated with cancer. In particular, the invention concerns prognostic methods based on the molecular characterization of gene expression in paraffin-embedded, fixed samples of cancer tissue, which allow a physician to predict whether a patient is likely to respond well to treatment with an EGFR inhibitor.

## Description

### Field of the Invention

The present invention concerns gene expression profiling of tissue samples obtained from patients who are candidates for treatment with a therapeutic EGFR inhibitor. More specifically, the invention provides methods based on the molecular characterization of gene expression in paraffin-embedded, fixed cancer tissue samples, which allow a physician to predict whether a patient is likely to respond well to treatment with an EGFR inhibitor.

### Description of the Related Art

Oncologists have a number of treatment options available to them, including different combinations of chemotherapeutic drugs that are characterized as "standard of care," and a number of drugs that do not carry a label claim for particular cancer, but for which there is evidence of efficacy in that cancer. Best likelihood of good treatment outcome requires that patients be assigned to optimal available cancer treatment, and that this assignment be made as quickly as possible following diagnosis.

Currently, diagnostic tests used in clinical practice are single analyte, and therefore do not capture the potential value of knowing relationships between dozens of different markers. Moreover, diagnostic tests are frequently not quantitative, relying on immunohistochemistry. This method often yields different results in different laboratories, in part because the reagents are not standardized, and in part because the interpretations are subjective and cannot be easily quantified. RNA-based tests have not often been used because of the problem of RNA degradation over time and the fact that it is difficult to obtain fresh tissue samples from patients for analysis. Fixed paraffin-embedded tissue is more readily available. Fixed tissue has been routinely used for non-quantitative detection of RNA, by in situ hybridization. However, recently methods have been established to quantify RNA in fixed tissue, using RT-PCR. This technology platform can also form the basis for multi-analyte assays

Recently, several groups have published studies concerning the classification of various cancer types by microarray gene expression analysis (see, e.g. Golub et al., Science 286:531-537 (1999); Bhattacharjae et al., Proc. Natl. Acad. Sci. USA 98:13790-13795 (2001); Chen-Hsiang et al., Bioinformatics 17 (Suppl. 1):S316-S322 (2001); Ramaswamy et al., Proc. Natl. Acad. Sci. USA 98:15149-15154 (2001)). Certain classifications of human breast cancers based on gene expression patterns have also been reported (Martin et al., Cancer Res. 60:2232-2238 (2000); West et al., Proc. Natl. Acad. Sci. USA 98:11462-11467 (2001); Sorlie et al., Proc. Natl. Acad. Sci. USA 98:10869-10874 (2001); Yan et al., Cancer Res. 61:8375-8380 (2001)). However, these studies mostly focus on improving and refining the already established classification of various types of cancer, including breast cancer, and generally do not link the findings to treatment strategies in order to improve the clinical outcome of cancer therapy.

Although modem molecular biology and biochemistry have revealed hundreds of genes whose activities influence the behavior of tumor cells, the state of their differentiation, and their sensitivity or resistance to certain therapeutic drugs, with a few exceptions, the status of these genes has not been exploited for the purpose of routinely making clinical decisions about drug treatments. One notable exception is the use of estrogen receptor (ER) protein expression in breast carcinomas to select patients to treatment with anti-estrogen drugs, such as tamoxifen. Another exceptional example is the use of ErbB2 (Her2) protein expression in breast carcinomas to select patients with the Her2 antagonist drug Herceptin® (Genentech, Inc., South San Francisco, CA).

Despite recent advances, a major challenge in cancer treatment remains to target specific treatment regimens to pathogenically distinct tumor types, and ultimately personalize tumor treatment in order to optimize outcome. Hence, a need exists for tests that simultaneously provide predictive information about patient responses to the variety of treatment options.

### Summary of the Invention

The present invention is based on findings of a Phase II clinical study of gene expression in tissue samples obtained from human patients with non-small cell lung cancer (NSCLC) who responded or did not respond to treatment with EGFR inhibitors.

In one aspect, the invention concerns a method for predicting the likelihood that a cancer patient who is a candidate for treatment with a therapeutic EGFR inhibitor will respond to treatment with an EGFR inhibitor, comprising determining the expression level of one or more prognostic RNA transcripts or their expression products in a biological sample comprising tumor cells, such as a tumor tissue specimen, obtained from the patient, wherein the prognostic transcript is the transcript of one or more genes selected from the group consisting of:
hCRAa; LAMC2; B2M; STAT5B; LMYC; CKAP4; TAGLN; Furin; DHFR; CCND3; TITF1; FUS; FLT1; TIMP2; RASSF1; WISP1; VEGFC; GPX2; CTSH; AKAP12; APC; RPL19; IGFBP6; Bak; CyclinG1; Hepsin1; MMP2; XIAP; MUC1; STMY3; PDGFRb; GSTp; p53R2; DPYD; IGFBP3; MMP9; RRM; KRT17; PDGFRa; EPHX1; E2F1; HNF3A; mGST1; STAT3; IGF1R; EGFR; cdc25A; RPLPO; YB-1; CKAP4; Kitlng; HER2; Surfact A; BTC; PGK1; MTA1; FOLR1; Claudin 4, EMP1, wherein
   (a) increased expression of one or more of hCRAa; LAMC2; STAT5B; CKAP4; TAGLN; Furin; FUS; FLT1; TIMP2; RASSF1; WISP1; VEGFC; GPX2; AKAP12; RPL19; IGFBP6; MMP2; STMY3; PDGFRb; GSTp; IGFBP3; MMP9; KRT17; PDGFRa; IGF1R; cdc25A; RPLPO; YB-1; CKAP4, EMP1 or the corresponding expression product, indicates that the patient is not likely to respond well to treatment with an EGFR inhibitor, and
   (b) increased expression of one or more of B2M; LMYC; DHFR; CCND3; TITF1; CTSH; APC; Bak; CyclinG1; Hepsin1; XIAP; MUC1; p53R2; DPYD; RRM; EPHX1; E2F1; HNF3A; mGST1; STAT3; EGFR; Kitlng; HER2; Surfact A; BTC; PGK1; MTA1; FOLR1; Claudin 4, or the corresponding gene product, indicates that the patient is likely to respond well to treatment with an EGFR inhibitor.

The tissue sample preferably is a fixed, paraffin-embedded tissue. Tissue can be obtained by a variety of methods, including fine needle, aspiration, bronchial lavage, or transbronchial biopsy.

In a specific embodiment, the expression level of the prognostic RNA transcript or transcripts is determined by RT-PCR. In this case, and when the tissue sample is fixed, and paraffin-embedded, the RT-PCR amplicons (defined as the polynucleotide sequence spanned by the PCR primers) should preferably be less than 100 bases in length. In other embodiments, the levels of the expression product of the prognostic RNA transcripts are determined by other methods known in the art, such as immunohistochemistry, or proteomics technology. The assays for measuring the prognostic RNA transcripts or their expression products may be available in a kit format.

In another aspect, the invention concerns an array comprising polynucleotides hybridizing to one or more of the following genes: hCRA a; LAMC2; B2M; STAT5B; LMYC; CKAP4; TAGLN; Furin; DHFR; CCND3; TITF1; FUS; FLT1; TIMP2; RASSF1; WISP1; VEGFC; GPX2; CTSH; AKAP12; APC; RPL19; IGFBP6; Bak; CyclinG1; Hepsin1; MMP2; XIAP; MUC1; STMY3; PDGFRb; GSTp; p53R2; DPYD; IGFBP3; MMP9; RRM; KRT17; PDGFRa; EPHX1; E2F1; HNF3A; mGST1; STAT3; IGF1R; EGFR; cdc25A; RPLPO; YB-1; CKAP4; Kitlng; HER2; Surfact A; BTC; PGK1; MTA1; FOLR1; Claudin 4; EMP1, immobilized on a solid surface. The polynucleotides can be cDNA or oligonucleotides. The cDNAs are typically about 500 to 5000 bases long, while the oligonucleotides are typically about 20 to 80 bases long. An array can contain a very large number of cDNAs, or oligonucleotides, e.g. up to about 330,000 oligonucleotides. The solid surface presenting the array can, for example, be glass. The levels of the product of the gene transcripts can be measured by any technique known in the art, including, for example, immunohistochemistry or proteomics.

In various embodiments, the array comprises polynucleotides hybridizing to two at least two, at least three, at least four, at least five, at least six, at least seven, at least eight, at least nine, at least ten, at least eleven, at least twelve, at least thirteen, at least fourteen, at least fifteen, at least seventeen, at least eighteen, at least nineteen, at least twenty, at least twenty-one, at least twenty-two, at least twenty-three, at least twenty-four, at least twenty-five, at least twenty-six, or all twenty-seven of the genes listed above. In a particular embodiment, hybridization is performed under stringent conditions.

In other embodiments, the array may comprise more than one polynucleotide hybridizing to the same gene.

In yet another embodiment, the array may comprise intron-based sequences, the expression of which correlated with the expression of a corresponding exon. Arrays comprising such intron-based sequences are disclosed, for example, in copending application Serial No. 10/783,884 filed on February 19, 2004, and in its PCT counterpart PCT/US04/05287 filed on February 19, 2004.

The invention further concerns a method of preparing a personalized genomics profile for a patient, comprising the steps of:
(a) subjecting RNA extracted from cancer tissue obtained from the patient to gene expression analysis;
(b) determining the expression level in the tissue of one or more genes selected from the group consisting of hCRA a; LAMC2; B2M; STAT5B; LMYC; CKAP4; TAGLN; Furin; DHFR; CCND3; TITF1; FUS; FLT1; TIMP2; RASSF1; WISP1; VEGFC; GPX2; CTSH; AKAP12; APC; RPL19; IGFBP6; Bak; CyclinG1; Hepsin1; MMP2; XIAP; MUC1; STMY3; PDGFRb; GSTp; p53R2; DPYD; IGFBP3; MMP9; RRM; KRT17; PDGFRa; EPHX1; E2F1; HNF3A; mGST1; STAT3; IGF1R; EGFR; cdc25A; RPLPO; YB-1; CKAP4; Kitlng; HER2; Surfact A; BTC; PGK1; MTA1; FOR1; Claudin 4; EMP1, wherein the expression level is normalized against a control gene or genes and optionally is compared to the amount found in a corresponding cancer reference tissue set; and
(c) creating a report summarizing the data obtained by said gene expression analysis.

The report may include treatment recommendations, and the method may comprise a step of treating the patient following such treatment recommendations.

The invention additionally concerns a method for amplification of a gene selected from the group consisting of hCRA a; LAMC2; B2M; STAT5B; LMYC; CKAP4; TAGLN; Furin; DHFR; CCND3; TITF1; FUS; FLT1; TIMP2; RASSF1; WISP1; VEGFC; GPX2; CTSH; AKAP12; APC; RPL19; IGFBP6; Bak; CyclinG1; Hepsin1; MMP2; XIAP; MUC1; STMY3; PDGFRb; GSTp; p53R2; DPYD; IGFBP3; MMP9; RRM; KRT17; PDGFRa; EPHX1; E2F1; HNF3A; mGST1; STAT3; IGF1R; EGFR; cdc25A; RPLPO; YB-1; CKAP4; Kitlng; HER2; Surfact A; BTC; PGK1; MTA1; FOR1; Claudin 4; EMP1 by polymerase chain reaction (PCR), comprising performing said PCR by using a corresponding amplicon listed in Table 3, and a corresponding primer-probe set listed in Table 4.

The invention further encompasses any PCR primer-probe set listed in Table 4 and any PCR amplicon listed in Table 3.

### Brief Description of the Drawings

Table 1 is a list of genes, expression of which correlates, positively or negatively, with patient response to treatment with an EGFR inhibitor.
Table 2 shows the results of binary statistical analysis of a list of genes, expression of which correlates with patient response to treatment with an EGFR inhibitor.
Table 3 is a list of genes, expression of which predict patient response to treatment with an EGFR inhibitor. The table includes accession numbers for the genes, and sequences for the forward and reverse primers (designated by "f' and "r", respectively) and probes (designated by "p") used for PCR amplification.
Table 4 shows the amplicon sequences used in PCR amplification of the indicated genes.

### Detailed Description of the Preferred Embodiment

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single-and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically cancer, such as breast cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The term "over-expression" with regard to an RNA transcript is used to refer the level of the transcript determined by normalization to the level of reference mRNAs, which might be all measured transcripts in the specimen or a particular reference set of mRNAs.

The phrase "gene amplification" refers to a process by which multiple copies of a gene or gene fragment are formed in a particular cell or cell line. The duplicated region (a stretch of amplified DNA) is often referred to as "amplicon." Usually, the amount of the messenger RNA (mRNA) produced, *i*.*e*., the level of gene expression, also increases in the proportion of the number of copies made of the particular gene expressed.

The term "prognosis" is used herein to refer to the prediction of the likelihood of cancer-attributable death or progression, including recurrence, metastatic spread, and drug resistance, of a neoplastic disease, such as non-small cell lung cancer, or head and neck cancer. The term "prediction" is used herein to refer to the likelihood that a patient will respond either favorably or unfavorably to a drug or set of drugs, and also the extent of those responses, or that a patient will survive, following surgical removal or the primary tumor and/or chemotherapy for a certain period of time without cancer recurrence. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as surgical intervention, chemotherapy with a given drug or drug combination, and/or radiation therapy, or whether long-term survival of the patient, following surgery and/or termination of chemotherapy or other treatment modalities is likely.

The term "long-term" survival is used herein to refer to survival for at least 1 year, more preferably for at least 2 years, most preferably for at least 5 years following surgery or other treatment.

The term "increased resistance" to a particular drug or treatment option, when used in accordance with the present invention, means decreased response to a standard dose of the drug or to a standard treatment protocol.

The term "decreased sensitivity" to a particular drug or treatment option, when used in accordance with the present invention, means decreased response to a standard dose of the drug or to a standard treatment protocol, where decreased response can be compensated for (at least partially) by increasing the dose of drug, or the intensity of treatment.

"Patient response" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

The term "treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. In tumor (*e.g*., cancer) treatment, a therapeutic agent may directly decrease the pathology of tumor cells, or render the tumor cells more susceptible to treatment by other therapeutic agents, *e.g*., radiation and/or chemotherapy.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, and brain cancer.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

The term "EGFR inhibitor" as used herein refers to a molecule having the ability to inhibit a biological function of a native epidermal growth factor receptor (EGFR). Accordingly, the term "inhibitor" is defined in the context of the biological role of EGFR. While preferred inhibitors herein specifically interact with (e.g. bind to) an EGFR, molecules that inhibit an EGFR biological activity by interacting with other members of the EGFR signal transduction pathway are also specifically included within this definition. A preferred EGFR biological activity inhibited by an EGFR inhibitor is associated with the development, growth, or spread of a tumor. EGFR inhibitors, without limitation, include peptides, non-peptide small molecules, antibodies, antibody fragments, antisense molecules, and oligonucleotide decoys.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like. In the context of the present invention, reference to "at least one," "at least two," "at least five," etc. of the genes listed in any particular gene set means any one or any and all combinations of the genes listed.

In the context of the present invention, reference to "at least one," "at least two," "at least five," etc. of the genes listed in any particular gene set means any one or any and all combinations of the genes listed.

The term "normalized" with regard to a gene transcript or a gene expression product refers to the level of the transcript or gene expression product relative to the mean levels of transcripts/products of a set of reference genes, wherein the reference genes are either selected based on their minimal variation across, patients, tissues or treatments ("housekeeping genes"), or the reference genes are the totality of tested genes. In the latter case, which is commonly referred to as "global normalization", it is important that the total number of tested genes be relatively large, preferably greater than 50. Specifically, the term 'normalized' with respect to an RNA transcript refers to the transcript level relative to the mean of transcript levels of a set of reference genes. More specifically, the mean level of an RNA transcript as measured by TaqMan® RT-PCR refers to the Ct value minus the mean Ct values of a set of reference gene transcripts.

The terms "expression threshold," and "defined expression threshold" are used interchangeably and refer to the level of a gene or gene product in question above which the gene or gene product serves as a predictive marker for patient response or resistance to a drug. The threshold typically is defined experimentally from clinical studies. The expression threshold can be selected either for maximum sensitivity (for example, to detect all responders to a drug), or for maximum selectivity (for example to detect only responders to a drug), or for minimum error.

### B. Detailed Description

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

### 1. Gene Expression Profiling

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS).

### 2. PCR-based Gene Expression Profiling Methods

### a. Reverse Transcriptase PCR (RT-PCR)

One of the most sensitive and most flexible quantitative PCR-based gene expression profiling methods is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, head and neck, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700^{™} Sequence Detection System^{™} (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700^{™} Sequence Detection System^{™}. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a relatively constant level among different tissues, and is unaffected by the experimental treatment. RNAs frequently used to normalize patterns of gene expression are mRNAs for the housekeeping genes glyceraldehyde-3-phosphate-dehydrogenase (GAPDH) and β-actin.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for profiling gene expression using fixed, paraffin-embedded tissues as the RNA source, including mRNA isolation, purification, primer extension and amplification are given in various published journal articles {for example: T.E. Godfrey et al. J. Molec. Diagnostics 2: 84-91 [2000]; K. Specht et al., Am. J. Pathol. 158: 419-29 [2001]}. Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR.

### b. MassARRAY System

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### c. Other PCR-based Methods

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex¹⁰⁰ LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### 3. Microarrays

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Agilent"s microarray technology.

The development of microarray methods for large-scale analysis of gene expression makes it possible to search systematically for molecular markers of cancer classification and outcome prediction in a variety of tumor types.

### 4. Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### 5. Gene Expression Analysis by Massively Parallel Signature Sequencing (MPSS)

This method, described by Brenner et al., Nature Biotechnology 18:630-634 (2000), is a sequencing approach that combines non-gel-based signature sequencing with *in vitro* cloning of millions of templates on separate 5 µm diameter microbeads. First, a microbead library of DNA templates is constructed by *in vitro* cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

### Immunohistochemistry

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

### Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

### 8. General Description of mRNA Isolation, Purification and Amplification

The steps of a representative protocol for profiling gene expression using fixed, paraffin-embedded tissues as the RNA source, including mRNA isolation, purification, primer extension and amplification are given in various published journal articles (for example: T.E. Godfrey et al. J. Molec. Diagnostics 2: 84-91 [2000]; K. Specht et al., Am. J. Pathol. 158: 419-29 [2001]). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR. Finally, the data are analyzed to identify the best treatment option(s) available to the patient on the basis of the characteristic gene expression pattern identified in the tumor sample examined.

### 9. EGFR Inhibitors

The epidermal growth factor receptor (EGFR) family (which includes EGFR, erb-B2, erb-B3, and erb-B4) is a family of growth factor receptors that are frequently activated in epithelial malignancies. Thus, the epidermal growth factor receptor (EGFR) is known to be active in several tumor types, including, for example, ovarian cancer, pancreatic cancer, non-small cell lung cancer {NSCLC}, breast cancer, and head and neck cancer. Several EGFR inhibitors, such as ZD1839 (also known as gefitinib or Iressa); and OSI774 (Erlotinib, Tarceva™), are promising drug candidates for the treatment of cancer.

Iressa, a small synthetic quinazoline, competitively inhibits the ATP binding site of EGFR, a growth-promoting receptor tyrosine kinase, and has been in Phase III clinical trials for the treatment of non-small-cell lung carcinoma. Another EGFR inhibitor, [agr]cyano-[bgr]methyl-*N*-[(trifluoromethoxy)phenyl]-propenamide (LFM-A12), has been shown to inhibit the proliferation and invasiveness of human breast cancer cells.

Cetuximab is a monoclonal antibody that blocks the EGFR and EGFR-dependent cell growth. It is currently being tested in phase III clinical trials.

Tarceva™ has shown promising indications of anti-cancer activity in patients with advanced ovarian cancer, and non-small cell lung and head and neck carcinomas.

The present invention provides valuable molecular markers that predict whether a patient who is a candidate for treatment with an EGFR inhibitor drug is likely to respond to treatment with an EGFR inhibitor.

The listed examples of EGFR inhibitors represent both small organic molecule and anti-EGFR antibody classes of drugs. The findings of the present invention are equally applicable to other EGFR inhibitors, including, without limitation, antisense molecules, small peptides, etc.

Further details of the invention will be apparent from the following non-limiting Example.

### Example

### A Phase II Study of Gene Expression in non-small cell lung cancer (NSCL)

A gene expression study was designed and conducted with the primary goal to molecularly characterize gene expression in paraffin-embedded, fixed tissue samples of NSCLC patients who did or did not respond to treatment with an EGFR inhibitor. The results are based on the use of one EGFR inhibitor.

### Study design

Molecular assays were performed on paraffin-embedded, formalin-fixed tumor tissues obtained from 39 individual patients diagnosed with NSCLC. Patients were included in the study only if histopathologic assessment, performed as described in the Materials and Methods section, indicated adequate amounts of tumor tissue. All patients had a history of prior treatment for NSCLC, and the nature of pretreatment varied.

### Materials and Methods

Each representative tumor block was characterized by standard histopathology for diagnosis, semi-quantitative assessment of amount of tumor, and tumor grade. A total of 6 sections (10 microns in thickness each) were prepared and placed in two Costar Brand Microcentrifuge Tubes (Polypropylene, 1.7 mL tubes, clear; 3 sections in each tube). If the tumor constituted less than 30% of the total specimen area, the sample may have been dissected by the pathologist, putting the tumor tissue directly into the Costar tube.

If more than one tumor block was obtained as part of the surgical procedure, the block most representative of the pathology was used for analysis.

### Gene Expression Analysis

mRNA was extracted and purified from fixed, paraffin-embedded tissue samples, and prepared for gene expression analysis as described above.

Molecular assays of quantitative gene expression were performed by RT-PCR, using the ABI PRISM 7900^{™} Sequence Detection System^{™} (Perkin-Elmer-Applied Biosystems, Foster City, CA, USA). ABI PRISM 7900^{™} consists of a thermocycler, laser, charge-coupled device (CCD), camera and computer. The system amplifies samples in a 384-well format on a thermocycler. During amplification, laser-induced fluorescent signal is collected in real-time through fiber optics cables for all 384 wells, and detected at the CCD. The system includes software for running the instrument and for analyzing the data.

### Analysis and Results

Tumor tissue was analyzed for 187 cancer-related genes and 5 reference genes. The threshold cycle (CT) values for each patient were normalized based on the mean of all genes for that particular patient. Clinical outcome data were available for all patients.

Outcomes were evaluated in two ways, each breaking patients into two groups with respect to response.

One analysis categorized complete or partial response [RES] as one group, and stable disease (min of 3 months) or progressive disease as the other group [NR]. The second analysis grouped patients with respect to clinical benefit, where clinical benefit was defined as partial response, complete response, or stable disease at 3 months.

Response (partial response and complete response) was determined by the Response Evaluation Criteria In Solid Tumors (RECIST criteria). Stable disease was designated as the absence of aggressive disease for 3 or more months.

### Analysis of patients by t-test

Analysis was performed on all 39 treated patients to determine the relationship between normalized gene expression and the binary outcomes of RES (response) or NR (non-response). A t test was performed on the group of patients classified as RES or NR and the p-values for the differences between the groups for each gene were calculated. The following table lists the 39 genes for which the p-value for the differences between the groups was <0.15. In this case response was defined as a partial or complete response, the former being >50% shrink of the tumor and the latter being disappearance of the tumor. As shown, response was identified in 7 patients.

**Table 1**

| | Mean No Response | Mean Response | P | Valid N No Response | Valid N Response |
|---|---|---|---|---|---|
| DHFR | -2.35 | -1.55 | 0.0095 | 32 | 7 |
| TITF1 | -4.64 | -2.53 | 0.0108 | 32 | 7 |
| B2M | -0.19 | 0.81 | 0.0126 | 32 | 7 |
| MUC1 | -1.13 | 0.49 | 0.0201 | 32 | 7 |
| XIAP | -3.63 | -2.98 | 0.0212 | 32 | 7 |
| Furin | -3.64 | -4.70 | 0.0333 | 32 | 7 |
| STAT5B | -2.21 | -2.71 | 0.0482 | 32 | 7 |
| RRM1 | -4.09 | -3.52 | 0.0484 | 32 | 7 |
| DPYD | -0.67 | -0.17 | 0.0509 | 32 | 7 |
| KRT17 | -4.02 | -5.90 | 0.0513 | 32 | 7 |
| PDGFRa | -1.92 | -3.16 | 0.0521 | 32 | 7 |
| TIMP2 | 1.51 | 0.89 | 0.0522 | 32 | 7 |
| EPHX1 | -1.23 | -0.31 | 0.0551 | 32 | 7 |
| Hepsin | -7.02 | -6.48 | 0.0617 | 32 | 7 |
| E2F1 | -5.09 | -4.28 | 0.0620 | 32 | 7 |
| HNF3A | -4.27 | -3.03 | 0.0688 | 32 | 7 |
| GPX2 | -4.65 | -6.30 | 0.0784 | 32 | 7 |
| mGST1 | -1.05 | -0.08 | 0.0872 | 32 | 7 |
| LAMC2 | -3.67 | -4.69 | 0.0874 | 32 | 7 |
| STAT3 | -0.01 | 0.42 | 0.1045 | 32 | 7 |
| IGF1R | -3.99 | -4.62 | 0.1051 | 32 | 7 |
| WISP1 | -5.23 | -5.97 | 0.1065 | 32 | 7 |
| p53R2 | -2.79 | -2.22 | 0.1125 | 32 | 7 |
| EGFR | -2.25 | -1.43 | 0.1151 | 32 | 7 |
| cdc25A | -5.40 | -5.92 | 0.1205 | 32 | 7 |
| RPLPO | 1.39 | 1.09 | 0.1217 | 32 | 7 |
| TAGLN | 0.58 | -0.51 | 0.1255 | 32 | 7 |
| YB-1 | 0.14 | -0.11 | 0.1257 | 32 | 7 |
| CKAP4 | -1.37 | -1.89 | 0.1262 | 32 | 7 |
| Kitlng | -3.62 | -2.86 | 0.1291 | 32 | 7 |
| HER2 | -2.22 | -1.33 | 0.1313 | 32 | 7 |
| hCRA a | -5.86 | -6.48 | 0.1332 | 32 | 7 |
| Surfact A1 | -1.00 | 2.24 | 0.1341 | 32 | 7 |
| LMYC | -4.62 | -4.20 | 0.1354 | 32 | 7 |
| BTC | -6.16 | -5.50 | 0.1390 | 32 | 7 |
| PGK1 | -1.18 | -0.75 | 0.1400 | 32 | 7 |
| MTA1 | -3.48 | -3.05 | 0.1451 | 32 | 7 |
| FOLR1 | -3.40 | -1.81 | 0.1455 | 32 | 7 |
| Claudin 4 | -1.66 | -0.94 | 0.1494 | 32 | 7 |

In the foregoing Table 1, lower mean expression Cₜ values indicate lower expression and, conversely, higher mean expression values indicate higher expression of a particular gene. Thus, for example, expression of the STAT5B gene was higher in patients who did not respond to EGFR inhibitor treatment than in patients that did respond to the treatment. Accordingly, elevated expression of STAT5B is an indication of poor outcome of treatment with an EGFR inhibitor. Phrasing it differently, if the STAT5B gene is over-expressed in a tissue simple obtained from the cancer of a NSCLC patient, treatment with an EGFR inhibitor is not likely to work, therefore, the physician is well advised to look for alternative treatment options.

Accordingly, the elevated expression of Furin; STAT5B; KRT17; PDGFRa; TIMP2; GPX2; LAMC2; IGF1R; WISP1; cdc25A; RPLPO; TAGLN; YB-1; CKAP4; or hCRA in a tumor is an indication that the patient is not likely to respond well to treatment with an EGFR inhibitor. On the other hand, elevated expression of DHFR; TITF1; B2M; MLTC1; XIAP; RRM; DPYD; EPHX1; Hepsin; E2F1; HNF3A; most1; STAT3; p53R2; EGFR; Kitlng; HER2; Surfact A; LMYC; BTC; PGK1; MTA1; FOLR1, or Claudin 4 is an indication that the patient is likely to respond to EGFR inhibitor treatment.

In Table 2 below the binary analysis was carried with respect to clinical benefit, defined as either partial response, complete response, or stable disease. As shown, 12 patients met these criteria for clinical benefit.

**Table 2**

| | Mean | Mean | p | Valid N | Valid N |
|---|---|---|---|---|---|
| | No Benefit | Benefit | | No Benefit | Benefit |
| hCRA a | -5.63 | -6.75 | 0.0005 | 27 | 12 |
| LAMC2 | -3.40 | -4.88 | 0.0017 | 27 | 12 |
| B2M | -0.32 | 0.68 | 0.0022 | 27 | 12 |
| STAT5B | -2.15 | -2.65 | 0.0133 | 27 | 12 |
| LMYC | -4.72 | -4.16 | 0.0156 | 27 | 12 |
| CKAP4 | -1.27 | -1.89 | 0.0271 | 27 | 12 |
| TAGLN | 0.77 | -0.48 | 0.0305 | 27 | 12 |
| Furin | -3.56 | -4.44 | 0.0341 | 27 | 12 |
| DHFR | -2.37 | -1.84 | 0.0426 | 27 | 12 |
| CCND3 | -3.76 | -3.06 | 0.0458 | 27 | 12 |
| TITF1 | -4.69 | -3.30 | 0.0462 | 27 | 12 |
| FUS | -2.15 | -2.56 | 0.0496 | 27 | 12 |
| FLT1 | -6.01 | -6.58 | 0.0501 | 27 | 12 |
| TIMP2 | 1.55 | 1.05 | 0.0583 | 27 | 12 |
| RASSF1 | -3.23 | -3.64 | 0.0619 | 27 | 12 |
| WISP1 | -5.15 | -5.85 | 0.0657 | 27 | 12 |
| VEGFC | -7.09 | -7.35 | 0.0738 | 27 | 12 |
| GPX2 | -4.52 | -5.91 | 0.0743 | 27 | 12 |
| CTSH | -0.71 | 0.20 | 0.0743 | 27 | 12 |
| AKAP12 | -2.32 | -3.26 | 0.0765 | 27 | 12 |
| APC | -3.19 | -2.77 | 0.0792 | 27 | 12 |
| RPL19 | 2.06 | 1.75 | 0.0821 | 27 | 12 |
| IGFBP6 | -3.86 | -4.79 | 0.0920 | 27 | 12 |
| Bak | -4.01 | -3.65 | 0.0985 | 27 | 12 |
| Cyclin G1 | -7.18 | -7.01 | 0.0997 | 27 | 12 |
| Hepsin | -7.04 | -6.65 | 0.1067 | 27 | 12 |
| MMP2 | 0.28 | -0.77 | 0.1080 | 27 | 12 |
| XIAP | -3.63 | -3.25 | 0.1161 | 27 | 12 |
| MUC1 | -1.12 | -0.20 | 0.1198 | 27 | 12 |
| STMY3 | -2.67 | -3.67 | 0.1246 | 27 | 12 |
| PDGFRb | -2.26 | -3.01 | 0.1300 | 27 | 12 |
| GSTp | 0.48 | 0.05 | 0.1335 | 27 | 12 |
| p53R2 | -2.82 | -2.38 | 0.1337 | 27 | 12 |
| DPYD | -0.67 | -0.36 | 0.1385 | 27 | 12 |
| IGFBP3 | -1.61 | -2.31 | 0.1399 | 27 | 12 |
| MMP9 | -3.29 | -4.07 | 0.1497 | 27 | 12 |

As shown in the above Table 2, 6 genes correlated with clinical benefit with p<0.1. Expression of hCRA a; LAMC2; STAT5B; CKAP4; TAGLN; Furin; FUS; FLT1; TIMP2; RASSF1; WISP1; VEGFC; GPX2; AKAP12; RPL19; IGFBP6; MMP2; STMY3; PDGFRb; GSTp; IGFBP3; or MMP9 was higher in patients who did not respond to anti-EGFR treatment. Thus, greater expression of these genes is an indication that patients are unlikely to benefit from anti-EGFR treatment. Conversely, expression of B2M; LMYC; DHFR; CCND3; TITF1; CTSH; APC; Bak; CyclinG1; Hepsin1; XIAP; MUC1; p53R2, or DPYD was higher in patients who did respond to anti-EGFR treatment. Greater expression of these genes indicates that patients are likely to benefit from anti-EGFR treatment.

In addition to the above analysis, robust logistic regression (David W. Hosmer, Jr. and Stanley Lameshow [2000] Applied Logistic Regression, Wiley, N.Y; Peter J. Huber [1981] Robust Statistics, John Wiley &Sons, N.Y.).was performed to assess the relationship between response and EMP1 reference normalized gene expression level. A robust logistic estimation procedure based on Hubers M-estimate² was used to obtain an estimate of the probability of response as a function of EMP1 were obtained. Based on this analysis, it is estimated that a patient has less than a 10% probability of response for reference normalized EMP1 gene expression levels greater than -1.43. Therefore increased expression of the gene EMP1 decreases the likelihood of response to chemotherapy.

It is emphasized that while the data presented herein were obtained using tissue samples from NSCLC, the conclusions drawn from the tissue expression profiles are equally applicable to other cancers, such as, for example, colon cancer, ovarian cancer, pancreatic cancer, breast cancer, and head and neck cancer.

All references cited throughout the specification are hereby expressly incorporated by reference.

While the invention has been described with emphasis upon certain specific embodiments, it is be apparent to those skilled in the art that variations and modification in the specific methods and techniques are possible. Accordingly, this invention includes all modifications encompassed within the spirit and scope of the invention as defined by the following claims.

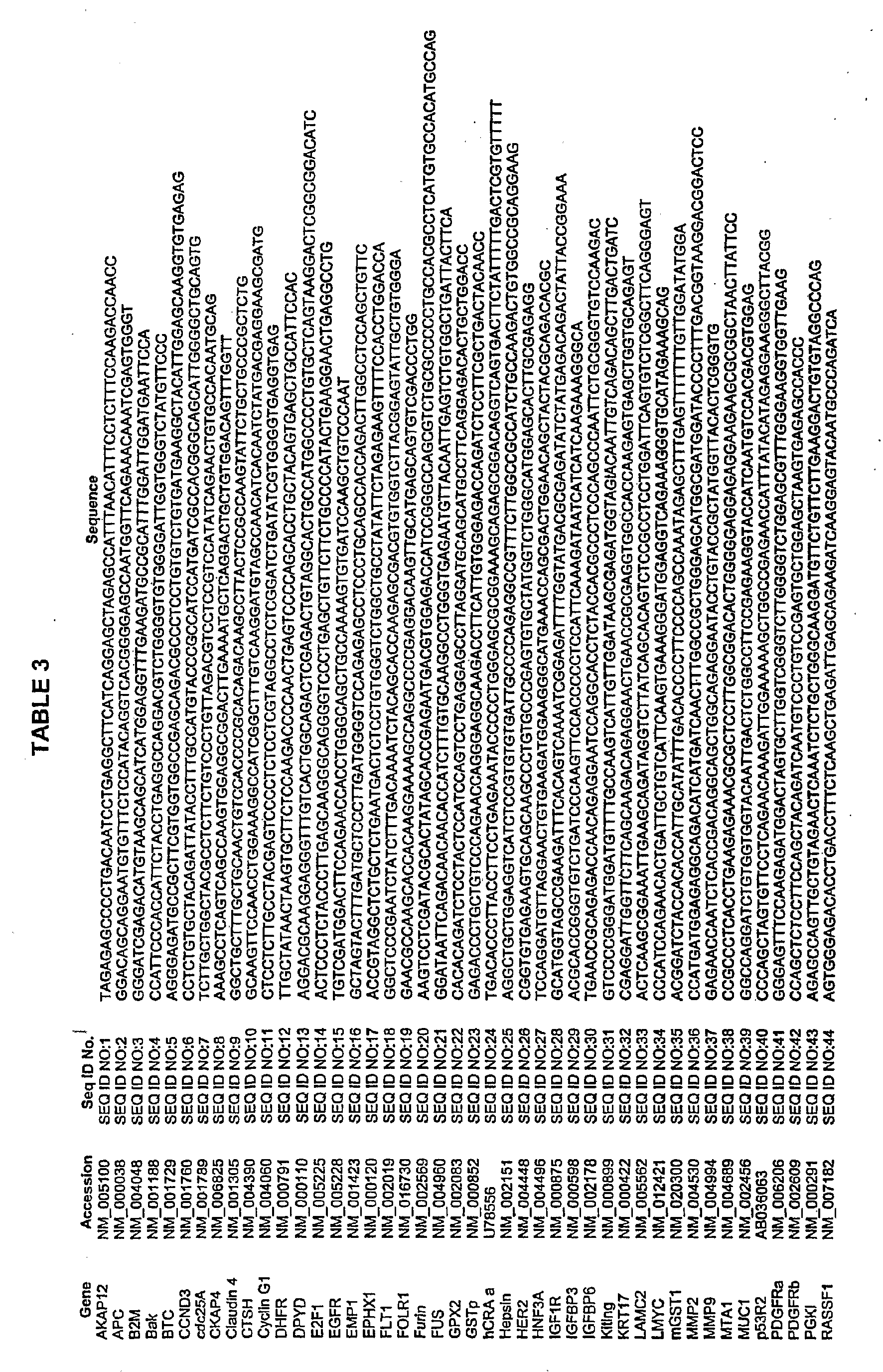

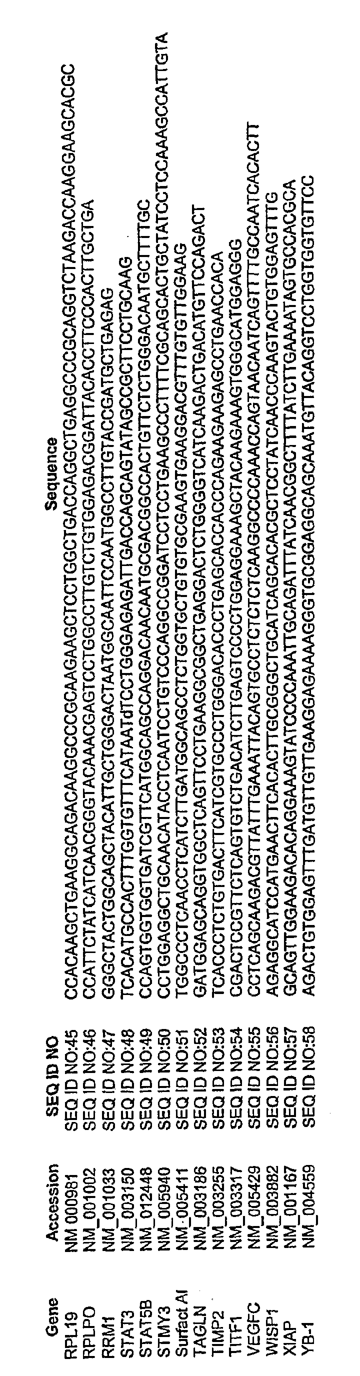

**TABLE 4**

| **Gene** | **Accession** | **Name** | **SEQ ID NO** | **Sequence** | **Length** |
|---|---|---|---|---|---|
| AKAP12 | NM_005100 | S3499/AKAP12.f2 | SEQ ID NO:59 | TAGAGAGCCCCTGACAATCC | 20 |
| AKAP12 | NM_005100 | S3500/AKAP12.r2 | SEQ ID NO:60 | GGTTGGTCTTGGAAAGAGGA | 20 |
| AKAP12 | NM_005100 | S3502IAKAP12.p2 | SEQ ID NO:61 | TGGCTCTAGCTCCTGATGAAGCCTC | 25 |
| APC | NM_000038 | S0022/APC.f4 | SEQ ID NO:62 | GGACAGCAGGAATGTGTTTC | 20 |
| APC | NM_000038 | S0024/APC.r4 | SEQ ID NO:63 | ACCCACTCGATTTGTTTCTG | 20 |
| APC | NM_000038 | S4888/APC.p4 | SEQ ID NO:64 | CATTGGCTCCCCGTGACCTGTA | 22 |
| B2M | NM_004048 | S1355/B2M.f4 | SEQ ID NO:65 | GGGATCGAGACATGTAAGCA | 20 |
| B2M | NM_004048 | S1356/B2M.r4 | SEQ ID NO:66 | TGGAATTCATCCAATCCAAAT | 21 |
| B2M | NM_004048 | S4932/B2M.p4 | SEQ ID NO:67 | CGGCATCTTCAAACCTCCATGATG | 24 |
| Bak | NM_001188 | S0037/Bak.f2 | SEQ ID NO:68 | CCATTCCCACCATTCTACCT | 20 |
| Bak | NM_001188 | S0039/Bak.r2 | SEQ ID NO:69 | GGGAACATAGACCCACCAAT | 20 |
| Bak | NM_001188 | S4724/Bak.p2 | SEQ ID NO:70 | ACACCCCAGACGTCCTGGCCT | 21 |
| BTC | NM_001729 | S1216/BTC.f3 | SEQ ID NO:71 | AGGGAGATGCCGCTTCGT | 18 |
| BTC | NM_001729 | S1217/BTC.r3 | SEQ ID NO:72 | CTCTCACACCTTGCTCCAATGTA | 23 |
| BTC | NM_001729 | S4844/BTC.p3 | SEQ ID NO:73 | CCTTCATCACAGACACAGGAGGGCG | 25 |
| CCND3 | NM_001760 | S2799/CCND3.f1 | SEQ ID NO:74 | CCTCTGTGCTACAGATTATACCTTTGC | 27 |
| CCND3 | NM_001760 | S2800/CCND3.r1 | SEQ ID NO:75 | CACTGCAGCCCCAATGCT | 18 |
| CCND3 | NM_001760 | S4966/CCND3.pl | SEQ ID NO:76 | TACCCGCCATCCATGATCGCCA | 22 |
| cdc25A | NM_001789 | S0070/cdc25A.f4 | SEQ ID NO:77 | TCTTGCTGGCTACGCCTCTT | 20 |
| cdc25A | NM_001789 | S0072/cdc25A.r4 | SEQ ID NO:78 | CTGCATTGTGGCACAGTTCTG | 21 |
| cdc25A | NM_001789 | S4989/cdc25A.p4 | SEQ ID NO:79 | TGTCCCTGTTAGACGTCCTCCGTCCATA | 28 |
| CKAP4 | NM_006825 | S2381/CKAP4.f2 | SEQ ID NO:80 | AAAGCCTCAGTCAGCCAAGT | 20 |
| CKAP4 | NM_006825 | S2382/CKAP4.r2 | SEQ ID NO:81 | AACCAAACTGTCCACAGCAG | 20 |
| CKAP4 | NM_006825 | S4892/CKAP4.p2 | SEQ ID NO:82 | TCCTGAGCATTTTCAAGTCCGCCT | 24 |
| Claudin 4 | NM_001305 | S2209/Claudi.f2 | SEQ ID NO:83 | GGCTGCTTTGCTGCAACTG | 19 |
| Claudin 4 | NM_001305 | S2210/Claudi.r2 | SEQ ID NO:84 | CAGAGCGGGCAGCAGAATA | 19 |
| Claudin 4 | NM_001305 | S4781/Claudi.p2 | SEQ ID NO:85 | CGCACAGACAAGCCTTACTCCGCC | 24 |
| CTSH | NM_004390 | S2363/CTSH.f2 | SEQ ID NO:86 | GCAAGTTCCAACCTGGAAAG | 20 |
| CTSH | NM_004390 | S2364/CTSH.r2 | SEQ ID NO:87 | CATCGCTTCCTCGTCATAGA | 20 |
| CTSH | NM_004390 | S4854/CTSH.p2 | SEQ ID NO:88 | TGGCTACATCCTTGACAAAGCCGA | 24 |
| Cyclin G1 | NM_004060 | S1946/Cyclin.f1 | SEQ ID NO:89 | CTCCTCTTGCCTACGAGTCC | 20 |
| Cyclin G1 | NM_004060 | S1947/Cyclin.r1 | SEQ ID NO:90 | CTCACCTCACCCCACGATA | 19 |
| Cyclin G1 | NM_004060 | S4755/Cyclin.p1 | SEQ ID NO:91 | CCTCTCCTCGTAGGCCTCTCGGAT | 24 |
| DHFR | NM_000791 | S0097/DHFR.f2 | SEQ ID NO:92 | TTGCTATAACTAAGTGCTTCTCCAAGA | 27 |
| DHFR | NM_000791 | S0099/DHFR.r2 | SEQ ID NO:93 | GTGGAATGGCAGCTCACTGTAG | 22 |
| DHFR | NM_000791 | S4997/DHFR.p2 | SEQ ID NO:94 | CCCAACTGAGTCCCCAGCACCT | 22 |
| DPYD | NM_000110 | SOIOO/DPYD.f2 | SEQ ID NO:95 | AGGACGCAAGGAGGGTTTG | 19 |
| DPYD | NM_000110 | S0102/DPYD.r2 | SEQ ID NO:96 | GATGTCCGCCGAGTCCTTACT | 21 |
| DPYD | NM_000110 | S4998/DPYD.p2 | SEQ ID NO:97 | CAGTGCCTACAGTCTCGAGTCTGCCAGTG | 29 |
| E2F1 | NM_005225 | S3063/E2F1.f3 | SEQ ID NO:98 | ACTCCCTCTACCCTTGAGCA | 20 |
| E2F1 | NM_005225 | S3064/E2F1.r3 | SEQ ID NO:99 | CAGGCCTCAGTTCCTTCAGT | 20 |
| E2F1 | NM_005225 | S4821/E2F1.p3 | SEQ ID NO:100 | CAGAAGAACAGCTCAGGGACCCCT | 24 |
| EGFR | NM_005228 | S0103/EGFR.f2 | SEQ ID NO:101 | TGTCGATGGACTTCCAGAAC | 20 |
| EGFR | NM_005228 | S0105/EGFR.r2 | SEQ ID NO:102 | ATTGGGACAGCTTGGATCA | 19 |
| EGFR | NM_005228 | S4999/EGFR.p2 | SEQ ID NO:103 | CACCTGGGCAGCTGCCAA | 18 |
| EMP1 | NM_001423 | S2796/EMPI.f1 | SEQ ID NO:104 | GCTAGTACTTTGATGCTCCCTTGAT | 25 |
| EMP1 | NM_001423 | S2797/EMP1.r1 | SEQ ID NO:105 | GAACAGCTGGAGGCCAAGTC | 20 |
| EMP1 | NM_001423 | S4964/EMP1.p1 | SEQ ID NO:106 | CCAGAGAGCCTCCCTGCAGCCA | 22 |
| EPHX1 | NM_000120 | S1865/EPHX1.f2 | SEQ ID NO:107 | ACCGTAGGCTCTGCTCTGAA | 20 |
| EPHX1 | NM_000120 | S1866/EPHX1.r2 | SEQ ID NO:108 | TGGTCCAGGTGGAAAACTTC | 20 |
| EPHX1 | NM_000120 | S4754/EPHX1.p2 | SEQ ID NO:109 | AGGCAGCCAGACCCACAGGA | 20 |
| FLT1 | NM_002019 | S1732/FLT1.f3 | SEQ ID NO:110 | GGCTCCCGAATCTATCTTTG | 20 |
| FLT1 | NM_002019 | S1733/FLT1.r3 | SEQ ID NO:111 | TCCCACAGCAATACTCCGTA | 20 |
| FLT1 | NM_002019 | S4922/FLT1.p3 | SEQ ID NO:112 | CTACAGCACCAAGAGCGACGTGTG | 24 |
| FOLR1 | NM_016730 | S2406/FOLR1.f1 | SEQ ID NO:113 | GAACGCCAAGCACCACAAG | 19 |
| FOLR1 | NM_016730 | S2407/FOLRI.r1 | SEQ ID NO:114 | CCAGGGTCGACACTGCTCAT | 20 |
| FOLR1 | NM_016730 | S4912/FOLR1.p1 | SEQ ID NO:115 | AAGCCAGGCCCCGAGGACAAGTT | 23 |
| Furin | NM_002569 | S2233/Furin.f1 | SEQ ID NO:116 | AAGTCCTCGATACGCACTATAGCA | 24 |
| Furin | NM_002569 | S2234/Furin.r1 | SEQ ID NO:117 | CTGGCATGTGGCACATGAG | 19 |
| Furin | NM_002569 | S4933/Furin.p1 | SEQ ID NO:118 | CCCGGATGGTCTCCACGTCAT | 21 |
| FUS | NM_004960 | S2936/FUS.f1 | SEQ ID NO:119 | GGATAATTCAGACAACAACACCATCT | 26 |
| FUS | NM_004960 | S2937/FUS.r1 | SEQ ID NO:120 | TGAAGTAATCAGCCACAGACTCAAT | 25 |
| FUS | NM_004960 | S4801/FUS.p1 | SEQ ID NO:121 | TCAATTGTAACATTCTCACCCAGGCCTTG | 29 |
| GPX2 | NM_002083 | S2514/GPX2.f2 | SEQ ID NO:122 | CACACAGATCTCCTACTCCATCCA | 24 |
| GPX2 | NM_002083 | S2515/GPX2.r2 | SEQ ID NO:123 | GGTCCAGCAGTGTCTCCTGAA | 21 |
| GPX2 | NM_002083 | S4936/GPX2.p2 | SEQ ID NO:124 | CATGCTGCATCCTAAGGCTCCTCAGG | 26 |
| GSTp | NM_000852 | S0136/GSTp.f3 | SEQ ID NO:125 | GAGACCCTGCTGTCCCAGAA | 20 |
| GSTp | NM_000852 | S0138/GSTp.r3 | SEQ ID NO:126 | GGTTGTAGTCAGCGAAGGAGATC | 23 |
| GSTp | NM_000852 | S5007/GSTp.p3 | SEQ ID NO:127 | TCCCACAATGAAGGTCTTGCCTCCCT | 26 |
| hCRA a | U78556 | S2198/hCRA a.f2 | SEQ ID NO:128 | TGACACCCTTACCTTCCTGAGAA | 23 |
| GSTp | NM_000852 | S5007/GSTp.p3 | SEQ ID NO:127 | TCCCACAATGAAGGTCTTGCCTCCCT | 26 |
| hCRA a | U78556 | S2198/hCRA a.f2 | SEQ ID NO:128 | TGACACCCTTACCTTCCTGAGAA | 23 |
| hCRA a | U78556 | S2199/hCRA a.r2 | SEQ ID NO:129 | AAAAACACGAGTCAAAAATAGAAGTCACT | 29 |
| hCRAa | U78556 | S4928/hCRA a.p2 | SEQ ID NO:130 | TCTGCTTTCCGCGCTCCCAGG | 21 |
| Hepsin | NM_002151 | S2269/Hepsin.f1 | SEQ ID NO:131 | AGGCTGCTGGAGGTCATCTC | 20 |
| Hepsin | NM_002151 | S2270/Hepsin.r1 | SEQ ID NO:132 | CTTCCTGCGGCCACAGTCT | 19 |
| Hepsin | NM_002151 | S4831/Hepsin.p1 | SEQ ID NO:133 | CCAGAGGCCGTTTCTTGGCCG | 21 |
| HER2 | NM_004448 | S0142/HER2.f3 | SEQ ID NO:134 | CGGTGTGAGAAGTGCAGCAA | 20 |
| HER2 | NM_004448 | S0144/HER2.r3 | SEQ ID NO:135 | CCTCTCGCAAGTGCTCCAT | 19 |
| HER2 | NM_004448 | S4729/HER2.p3 | SEQ ID NO:136 | CCAGACCATAGCACACTCGGGCAC | 24 |
| HNF3A | NM_004496 | S0148/HNF3A.f1 | SEQ ID NO:137 | TCCAGGATGTTAGGAACTGTGAAG | 24 |
| HNF3A | NM_004496 | S0150/HNF3A.r1 | SEQ ID NO:138 | GCGTGTCTGCGTAGTAGCTGTT | 22 |
| HNF3A | NM_004496 | S5008/HNF3A.p1 | SEQ ID NO:139 | AGTCGCTGGTTTCATGCCCTTCCA | 24 |
| IGFIR | NM_000875 | S1249/IGFIR.f3 | SEQ ID NO:140 | GCATGGTAGCCGAAGATTTCA | 21 |
| IGFIR | NM_000875 | S1250/IGFIR.r3 | SEQ ID NO:141 | TTTCCGGTAATAGTCTGTCTCATAGATATC | 30 |
| IGFIR | NM_000875 | S4895/IGFIR.p3 | SEQ ID NO:142 | CGCGTCATACCAAAATCTCCGATTTTGA | 28 |
| IGFBP3 | NM_000598 | S0157/IGFBP3.f3 | SEQ ID NO:143 | ACGCACCGGGTGTCTGA | 17 |
| IGFBP3 | NM_000598 | S0159/IGFBP3.r3 | SEQ ID NO:144 | TGCCCTTTCTTGATGATGATTATC | 24 |
| IGFBP3 | NM_000598 | S501 1/IGFBP3.p3 | SEQ ID NO:145 | CCCAAGTTCCACCCCCTCCATTCA | 24 |
| IGFBP6 | NM_002178 | S2335/IGFBP6.f1 | SEQ ID NO:146 | TGAACCGCAGAGACCAACAG | 20 |
| IGFBP6 | NM_002178 | S2336/IGFBP6.r1 | SEQ ID NO:147 | GTCTTGGACACCCGCAGAAT | 20 |
| IGFBP6 | NM_002178 | S4851/IGFBP6.p1 | SEQ ID NO:148 | ATCCAGGCACCTCTACCACGCCCTC | 25 |
| Kitlng | NM_000899 | S0169/Kitlng.f4 | SEQ ID NO:149 | GTCCCCGGGATGGATGTT | 18 |
| Kitlng | NM_000899 | S0171/Kitlng.r4 | SEQ ID NO:150 | GATCAGTCAAGCTGTCTGACAATTG | 25 |
| Kitlng | NM_000899 | S5012/Kitlng.p4 | SEQ ID NO:151 | CATCTCGCTTATCCAACAATGACTTGGCA | 29 |
| KRT17 | NM_000422 | S0172/KRT17.f2 | SEQ ID NO:152 | CGAGGATTGGTTCTTCAGCAA | 21 |
| KRT17 | NM_000422 | S0174/KRT17.r2 | SEQ ID NO:153 | ACTCTGCACCAGCTCACTGTTG | 22 |
| KRT17 | NM_000422 | S5013/KRT17.p2 | SEQ ID NO:154 | CACCTCGCGGTTCAGTTCCTCTGT | 24 |
| LAMC2 | NM_005562 | S2826/LAMC2.f2 | SEQ ID NO:155 | ACTCAAGCGGAAATTGAAGCA | 21 |
| LAMC2 | NM_005562 | S2827/LAMC2.r2 | SEQ ID NO:156 | ACTCCCTGAAGCCGAGACACT | 21 |
| LAMC2 | NM_005562 | S4969/LAMC2.p2 | SEQ ID NO:157 | AGGTCTTATCAGCACAGTCTCCGCCTCC | 28 |
| LMYC | NM_012421 | S2863/LMYC.f2 | SEQ ID NO:158 | CCCATCCAGAACACTGATTG | 20 |
| LMYC | NM_012421 | S2864/LMYC.r2 | SEQ ID NO:159 | CTGCTTTCTATGCACCCTTTC | 21 |
| LMYC | NM_012421 | S4973/LMYC.p2 | SEQ ID NO:160 | TGACCTCCATCCCTTTCACTTGAATG | 26 |
| mGST1 | NM_020300 | S2245/mGST1.f2 | SEQ ID NO:161 | ACGGATCTACCACACCATTGC | 21 |
| mGST1 | NM_020300 | S2246/mGST1.r2 | SEQ ID NO:162 | TCCATATCCAACAAAAAAACTCAAAG | 26 |
| mGST1 | NM_020300 | S4830/mGST1.p2 | SEQ ID NO:163 | TTTGACACCCCTTCCCCAGCCA | 22 |
| MMP2 | NM_004530 | S1874/MMP2.f2 | SEQ ID NO:164 | CCATGATGGAGAGGCAGACA | 20 |
| MMP2 | NM_004530 | S1875/MMP2.r2 | SEQ ID NO:165 | GGAGTCCGTCCTTACCGTCAA | 21 |
| MMP2 | NM_004530 | S5039/MMP2.p2 | SEQ ID NO:166 | CTGGGAGCATGGCGATGGATACCC | 24 |
| MMP9 | NM_004994 | S0656/MMP9.f1 | SEQ ID NO:167 | GAGAACCAATCTCACCGACA | 20 |
| MMP9 | NM_004994 | S0657/MMP9.r1 | SEQ ID NO:168 | CACCCGAGTGTAACCATAGC | 20 |
| MMP9 | NM_004994 | S4760/MMP9.p1 | SEQ ID NO:169 | ACAGGTATTCCTCTGCCAGCTGCC | 24 |
| MTA1 | NM_004689 | S2369/MTA1.f1 | SEQ ID NO:170 | CCGCCCTCACCTGAAGAGA | 19 |
| MTA1 | NM_004689 | S2370/MTA1.r1 | SEQ ID NO:171 | GGAATAAGTTAGCCGCGCTTCT | 22 |
| MTA1 | NM_004689 | S4855/MTA1.p1 | SEQ ID NO:172 | CCCAGTGTCCGCCAAGGAGCG | 21 |
| MUC1 | NM_002456 | S0782/MUC1.f2 | SEQ ID NO:173 | GGCCAGGATCTGTGGTGGTA | 20 |
| MUC1 | NM_002456 | S0783/MUC1.r2 | SEQ ID NO:174 | CTCCACGTCGTGGACATTGA | 20 |
| MUC1 | NM_002456 | S4807/MUC1.p2 | SEQ ID NO:175 | CTCTGGCCTTCCGAGAAGGTACC | 23 |
| p53R2 | AB036063 | S2305/p53R2.f3 | SEQ ID NO:176 | CCCAGCTAGTGTTCCTCAGA | 20 |
| p53R2 | AB036063 | S2306/p53R2.r3 | SEQ ID NO:177 | CCGTAAGCCCTTCCTCTATG | 20 |
| p53R2 | AB036063 | S4847/p53R2.p3 | SEQ ID NO:178 | TCGGCCAGCTTTTTCCAATCTTTG | 24 |
| PDGFRa | NM_006206 | S0226/PDGFRa.f2 | SEQ ID NO:179 | GGGAGTTTCCAAGAGATGGA | 20 |
| PDGFRa | NM_006206 | S0228/PDGFRa.r2 | SEQ ID NO:180 | CTTCAACCACCTTCCCAAAC | 20 |
| PDGFRa | NM_006206 | S5020/PDGFRa.p2 | SEQ ID NO:181 | CCCAAGACCCGACCAAGCACTAG | 23 |
| PDGFRb | NM_002609 | S1346/PDGFRb.f3 | SEQ ID NO:182 | CCAGCTCTCCTTCCAGCTAC | 20 |
| PDGFRb | NM_002609 | S1347/PDGFRb.r3 | SEQ ID NO:183 | GGGTGGCTCTCACTTAGCTC | 20 |
| PDGFRb | NM_002609 | S4931/PDGFRb.p3 | SEQ ID NO:184 | ATCAATGTCCCTGTCCGAGTGCTG | 24 |
| PGK1 | NM_000291 | S0232/PGK1.f1 | SEQ ID NO:185 | AGAGCCAGTTGCTGTAGAACTCAA | 24 |
| PGK1 | NM_000291 | S0234/PGK1.r1 | SEQ ID NO:186 | CTGGGCCTACACAGTCCTTCA | 21 |
| PGK1 | NM_000291 | S5022/PGK1.p1 | SEQ ID NO:187 | TCTCTGCTGGGCAAGGATGTTCTGTTC | 27 |
| RASSF1 | NM_007182 | S2393/RASSF1.f3 | SEQ ID NO:188 | AGTGGGAGACACCTGACCTT | 20 |
| RASSF1 | NM_007182 | S2394/RASSF1.r3 | SEQ ID NO:189 | TGATCTGGGCATTGTACTCC | 20 |
| RASSR | NM_007182 | S4909/RASSF1.p3 | SEQ ID NO:190 | TTGATCTTCTGCTCAATCTCAGCTTGAGA | 29 |
| RPL19 | NM_000981 | S0253/RPL19.f3 | SEQ ID NO:191 | CCACAAGCTGAAGGCAGACA | 20 |
| RPL19 | NM_000981 | S0255/RPL19.r3 | SEQ ID NO:192 | GCGTGCTTCCTTGGTCTTAGA | 21 |
| RPL19 | NM_000981 | S4728/RPL19.p3 | SEQ ID NO:193 | CGCAAGAAGCTCCTGGCTGACC | 22 |
| RPLPO | NM_001002 | S0256/RPLPO.f2 | SEQ ID NO:194 | CCATTCTATCATCAACGGGTACAA | 24 |
| RPLPO | NM_001002 | S0258/RPLPO.r2 | SEQ ID NO:195 | TCAGCAAGTGGGAAGGTGTAATC | 23 |
| RPLPO | NM_001002 | S4744/RPLPO.p2 | SEQ ID NO:196 | TCTCCACAGACAAGGCCAGGACTCG | 25 |
| RRM1 | NM_001033 | S2835/RRM1.f2 | SEQ ID NO:197 | GGGCTACTGGCAGCTACATT | 20 |
| RRM1 | NM_001033 | S2836/RRM1.r2 | SEQ ID NO:198 | CTCTCAGCATCGGTACAAGG | 20 |
| RRM1 | M_001033 | S4970/RRM1.p2 | SEQ ID NO:199 | CATTGGAATTGCCATTAGTCCCAGC | 25 |
| STAT3 | NM_003150 | S1545/STAT3.f1 | SEQ ID NO:200 | TCACATGCCACTTTGGTGTT | 20 |
| STAT3 | NM_003150 | S1546/STAT3.r1 | SEQ ID NO:201 | CTTGCAGGAAGCGGCTATAC | 20 |
| STAT3 | NM_003150 | S4881/STAT3.p1 | SEQ ID NO:202 | TCCTGGGAGAGATTGACCAGCA | 22 |
| STAT5B | NM_012448 | S2399/STAT5B.f2 | SEQ ID NO:203 | CCAGTGGTGGTGATCGTTCA | 20 |
| STAT5B | NM_012448 | S2400/STAT5B.r2 | SEQ ID NO:204 | GCAAAAGCATTGTCCCAGAGA | 21 |
| STAT5B | NM_012448 | S4910/STAT5B.p2 | SEQ ID NO:205 | CAGCCAGGACAACAATGCGACGG | 23 |
| STMY3 | NM_005940 | S2067/STMY3.f3 | SEQ ID NO:206 | CCTGGAGGCTGCAACATACC | 20 |
| STMY3 | NM_005940 | S2068/STMY3.r3 | SEQ ID NO:207 | TAGAATGGCTTTGGAGGATAGCA | 23 |
| STMY3 | NM_005940 | S4746/STMY3.p3 | SEQ ID NO:208 | ATCCTCCTGAAGCCCTTTTCGCAGC | 25 |
| Surfact A1 | NM_005411 | S2215/Surfac.f1 | SEQ ID NO:209 | TGGCCCTCAACCTCATCTTG | 20 |
| Surfact A1 | NM_005411 | S2216/Surfac.r1 | SEQ ID NO:210 | CTTCCAACACAAACGTCCTTCA | 22 |
| Surfact A1 | NM_005411 | S4930/Surfac.p1 | SEQ ID NO:211 | TTCGCACACAGCACCAGAGGCTG | 23 |
| TAGLN | NM_003186 | S3185/TAGLN.f3 | SEQ ID NO:212 | GATGGAGCAGGTGGCTCAGT | 20 |
| TAGLN | NM_003186 | S3186/TAGLN.r3 | SEQ ID NO:213 | AGTCTGGAACATGTCAGTCTTGATG | 25 |
| TAGLN | NM_003186 | S3266/TAGLN.p3 | SEQ ID NO:214 | CCCAGAGTCCTCAGCCGCCTTCAG | 24 |
| TIMP2 | NM_003255 | S1680/TIMP2.f1 | SEQ ID NO:215 | TCACCCTCTGTGACTTCATCGT | 22 |
| TIMP2 | NM_003255 | S1681/TIMP2.r1 | SEQ ID NO:216 | TGTGGTTCAGGCTCTTCTTCTG | 22 |
| TIMP2 | NM_003255 | S4916/TIMP2.p1 | SEQ ID NO:217 | CCCTGGGACACCCTGAGCACCA | 22 |
| TITF1 | NM_003317 | S2224/TITF1.f1 | SEQ ID NO:218 | CGACTCCGTTCTCAGTGTCTGA | 22 |
| TITF1 | NM_003317 | S2225/TITF1.r1 | SEQ ID NO:219 | CCCTCCATGCCCACTTTCT | 19 |
| TITF1 | NM_003317 | S4829/TITF1.p1 | SEQ ID NO:220 | ATCTTGAGTCCCCTGGAGGAAAGC | 24 |
| VEGFC | NM_005429 | S2251/VEGFC.f1 | SEQ ID NO:221 | CCTCAGCAAGACGTTATTTGAAATT | 25 |
| VEGFC | NM_005429 | S2252NEGFC.r1 | SEQ ID NO:222 | AAGTGTGATTGGCAAAACTGATTG | 24 |
| VEGFC | NM_005429 | S4758NEGFC.p1 | SEQ ID NO:223 | CCTCTCTCTCAAGGCCCCAAACCAGT | 26 |
| WISP1 | NM_003882 | S1671/WISPI.f1 | SEQ ID NO:224 | AGAGGCATCCATGAACTTCACA | 22 |
| WISP1 | NM_003882 | S1672/WISPI.r1 | SEQ ID NO:225 | CAAACTCCACAGTACTTGGGTTGA | 24 |
| WISP1 | NM_003882 | S4915/WISPI.p1 | SEQ ID NO:226 | CGGGCTGCATCAGCACACGC | 20 |
| XIAP | NM_001167 | S0289/XIAP.f1 | SEQ ID NO:227 | GCAGTTGGAAGACACAGGAAAGT | 23 |
| XIAP | NM_001167 | S0291/XIAP.r1 | SEQ ID NO:228 | TGCGTGGCACTATTTTCAAGA | 21 |
| XIAP | NM_001167 | S4752/XIAP.p1 | SEQ ID NO:229 | TCCCCAAATTGCAGATTTATCAACGGC | 27 |
| YB-1 | NM_004559 | S1194/YB-1.f2 | SEQ ID NO:230 | AGACTGTGGAGTTTGATGTTGTTGA | 25 |
| YB-1 | NM_004559 | S1195/YB-1.r2 | SEQ ID NO:231 | GGAACACCACCAGGACCTGTAA | 22 |
| YB-1 | NM_004559 | S4843/YB-1.p2 | SEQ ID NO:232 | TTGCTGCCTCCGCACCCTTTTCT | 23 |

## Claims

1. A method for predicting the likelihood that a patient will respond to treatment with an EGFR inhibitor, comprising
determining the normalized expression level of an RNA transcript of LAMC2, or its expression product, in a biological sample comprising cancer cells obtained from said patient, wherein increased expression of LAMC2, or its expression product, correlates to a decreased likelihood of response to treatment with an EGFR inhibitor.

2. The method of claim 1 wherein said subject is a human patient.

3. The method of claim 2 wherein said cancer is selected from the group consisting of ovarian cancer, colon cancer, pancreatic cancer, non-small cell lung cancer, breast cancer, and head and neck cancer.

4. The method of claim 1 wherein the biological sample is fixed, paraffin-embedded, or fresh, or frozen.

5. The method of claim 1 wherein the biological sample is obtained by fine needle, core, bronchial lavage, transbronchial, or other types of biopsy.

6. The method of claim 1 wherein the expression level of LAMC2 is determined by reverse transcription polymerase chain reaction (RT-PCR).

7. The method of claim 1 wherein the expression level of LAMC2 is determined by immunohistochemistry or other proteomics technology.

8. The method of claim 1 wherein the assay for measurement of LAMC2 is provided in the form of a kit or kits.

9. The method of claim 1 wherein the EGFR inhibitor is an antibody or an antibody fragment.

10. The method of claim 1 wherein the EGFR inhibitor is a small molecule.

11. An array comprising polynucleotides hybridizing to one of more of the following genes: LAMC2, hCRA a; B2M; STAT5B; LMYC; CKAP4; TAGLN; Furin; DHFR; CCND3; TITF1; FUS; FLT1; TIMP2; RASSF1; WISP1; VEGFC; GPX2; CTSH; AKAP12; APC; RPL19; IGFBP6; Bak; CyclinG1; Hepsin1; MMP2; XIAP; MUC1; STMY3; PDGFRb; GSTp; p53R2; DPYD; IGFBP3; MMP9; RRM; KRT17; PDGFRa; EPHX1; E2F1; HNF3A; mGST1; STAT3; IGF1R; EGFR; cdc25A; RPLPO; YB-1; CKAP4; Kiting; HER2; Surfact A; BTC; PGK1; MTA1; FOLR1; Claudin 4; EMP1, immobilized on a solid surface, wherein the array at least comprises polynucleotides hybridizing to LAMC2.

12. A method of preparing a personalized genomics profile for a patient, comprising the steps of:
(a) subjecting RNA extracted from cancer cells obtained from the patient to gene expression analysis;
(b) determining the expression level in the tissue of LAMC2, wherein the expression level is normalized against a control gene or genes and optionally is compared to the amount found in a corresponding cancer reference tissue set, and wherein increased expression of LAMC2 correlates to a decreased likelihood of response to treatment with an EGFR inhibitor; and
(c) creating a report summarizing the data obtained by said gene expression analysis.

13. The method of claim 12 wherein said solid tumor is selected from the group consisting of breast cancer, ovarian cancer, gastric cancer, colorectal cancer, pancreatic cancer, and lung cancer.

14. A method for amplification of LAMC2 by polymerase chain reaction (PCR), comprising performing said PCR by using a corresponding amplicon listed in Table 3, and a corresponding primer-probe set listed in Table 4.


SEQUENCE LISTING

<110> Genomic Health, Inc.
      Agus David
      Baker Joffre
      Natale Ron
      Shak Steven

<120> Gene Expression Markers for Response to
  EGFR Inhibitors Drugs


<130> 39740/0011

<140>       Not assigned
<141> 2004-05-30

<150> 60/474,908
<151> 2003-05-30

<160> 232

<170> FastSEQ for Windows Version 4.0

<210> 1
<211> 75
<212> DNA
<213> Artificial Sequence

<220>
<223> amplicon

<400> 1
tagagagccc ctgacaatcc tgaggcttca tcaggagcta gagccattta acatttcctc 60
tttccaagac caacc                                                  75

<210> 2
<211> 69
<212> DNA
<213> Artificial Sequence

<220>
<223> amplicon

<400> 2
ggacagcagg aatgtgtttc tccatacagg tcacggggag ccaatggttc agaaacaaat 60
cgagtgggt                                                         69

<210> 3
<211> 67
<212> DNA
<213> Artificial Sequence

<220>
<223> amplicon

<400> 3
gggatcgaga catgtaagca gcatcatgga ggtttgaaga tgccgcattt ggattggatg 60
aattcca                                                           67

<210> 4
<211> 66
<212> DNA
<213> Artificial Sequence

<220>

<223> amplicon

<400> 4
ccattcccac cattctacct gaggccagga cgtctggggt gtggggattg gtgggtctat 60
gttccc 66

<210> 5
<211> 81
<212> DNA
<213> Artificial Sequence

<220>
<223> amplicon

<400> 5
agggagatgc cgcttcgtgg tggccgagca gacgccctcc tgtgtctgtg atgaaggcta 60
cattggagca aggtgtgaga g 81

<210> 6
<211> 76
<212> DNA
<213> Artificial Sequence

<220>
<223> amplicon

<400> 6
cctctgtgct acagattata cctttgccat gtacccgcca tccatgatcg ccacgggcag 60
cattggggct gcagtg 76

<210> 7
<211> 71
<212> DNA
<213> Artificial Sequence

<220>
<223> amplicon

<400> 7
tcttgctggc tacgcctctt ctgtccctgt tagacgtcct ccgtccatat cagaactgtg 60
ccacaatgca g 71

<210> 8
<211> 66
<212> DNA
<213> Artificial Sequence

<220>
<223> amplicon

<400> 8
aaagcctcag tcagccaagt ggaggcggac ttgaaaatgc tcaggactgc tgtggacagt 60
ttggtt 66

<210> 9
<211> 72
<212> DNA
<213> Artificial Sequence

<220>
<223> amplicon

<400> 9
ggctgctttg ctgcaactgt ccaccccgca cagacaagcc ttactccgcc aagtattctg 60
ctgcccgctc tg 72

<210> 10

<211> 77
<212> DNA
<213> Artificial Sequence

<220>
<223> amplicon

<400> 10
gcaagttcca acctggaaag gccatcggct ttgtcaagga tgtagccaac atcacaatct 60
atgacgagga agcgatg 77

<210> 11
<211> 67
<212> DNA
<213> Artificial Sequence

<220>
<223> amplicon

<400> 11
ctcctcttgc ctacgagtcc cctctcctcg taggcctctc ggatctgata tcgtggggtg 60
aggtgag 67

<210> 12
<211> 73
<212> DNA
<213> Artificial Sequence

<220>
<223> amplicon

<400> 12
ttgctataac taagtgcttc tccaagaccc caactgagtc cccagcacct gctacagtga 60
gctgccattc cac 73

<210> 13
<211> 87
<212> DNA
<213> Artificial Sequence

<220>
<223> amplicon

<400> 13
aggacgcaag gagggtttgt cactggcaga ctcgagactg taggcactgc catggcccct 60
gtgctcagta aggactcggc ggacatc 87

<210> 14
<211> 75
<212> DNA
<213> Artificial Sequence

<220>
<223> amplicon

<400> 14
actccctcta cccttgagca agggcagggg tccctgagct gttcttctgc cccatactga 60
aggaactgag gcctg 75

<210> 15
<211> 62
<212> DNA
<213> Artificial Sequence

<220>
<223> amplicon

<400> 15
tgtcgatgga cttccagaac cacctgggca gctgccaaaa gtgtgatcca agctgtccca 60
at 62

<210> 16
<211> 75
<212> DNA
<213> Artificial Sequence

<220>
<223> amplicon

<400> 16
gctagtactt tgatgctccc ttgatggggt ccagagagcc tccctgcagc caccagactt 60
ggcctccagc tgttc 75

<210> 17
<211> 76
<212> DNA
<213> Artificial Sequence

<220>
<223> amplicon

<400> 17
accgtaggct ctgctctgaa tgactctcct gtgggtctgg ctgcctatat tctagagaag 60
ttttccacct ggacca 76

<210> 18
<211> 75
<212> DNA
<213> Artificial Sequence

<220>
<223> amplicon

<400> 18
ggctcccgaa tctatctttg acaaaatcta cagcaccaag agcgacgtgt ggtcttacgg 60
agtattgctg tggga 75

<210> 19
<211> 67
<212> DNA
<213> Artificial Sequence

<220>
<223> amplicon

<400> 19
gaacgccaag caccacaagg aaaagccagg ccccgaggac aagttgcatg agcagtgtcg 60
accctgg 67

<210> 20
<211> 95
<212> DNA
<213> Artificial Sequence

<220>
<223> amplicon

<400> 20
aagtcctcga tacgcactat agcaccgaga atgacgtgga gaccatccgg gccagcgtct 60
gcgccccctg ccacgcctca tgtgccacat gccag 95

<210> 21
<211> 80
<212> DNA

<213> Artificial Sequence

<220>
<223> amplicon

<400> 21
ggataattca gacaacaaca ccatctttgt gcaaggcctg ggtgagaatg ttacaattga 60
gtctgtggct gattacttca 80

<210> 22
<211> 75
<212> DNA
<213> Artificial Sequence

<220>
<223> amplicon

<400> 22
cacacagatc tcctactcca tccagtcctg aggagcctta ggatgcagca tgccttcagg 60
agacactgct ggacc 75

<210> 23
<211> 76
<212> DNA
<213> Artificial Sequence

<220>
<223> amplicon

<400> 23
gagaccctgc tgtcccagaa ccagggaggc aagaccttca ttgtgggaga ccagatctcc 60
ttcgctgact acaacc 76

<210> 24
<211> 90
<212> DNA
<213> Artificial Sequence

<220>
<223> amplicon

<400> 24
tgacaccctt accttcctga gaaatacccc ctgggagcgc ggaaagcaga gcggacaggt 60
cagtgacttc tatttttgac tcgtgttttt 90

<210> 25
<211> 84
<212> DNA
<213> Artificial Sequence

<220>
<223> amplicon

<400> 25
aggctgctgg aggtcatctc cgtgtgtgat tgccccagag gccgtttctt ggccgccatc 60
tgccaagact gtggccgcag gaag 84

<210> 26
<211> 70
<212> DNA
<213> Artificial Sequence

<220>
<223> amplicon

<400> 26
cggtgtgaga agtgcagcaa gccctgtgcc cgagtgtgct atggtctggg catggagcac 60

ttgcgagagg 70

<210> 27
<211> 73
<212> DNA
<213> Artificial Sequence

<220>
<223> amplicon

<400> 27
tccaggatgt taggaactgt gaagatggaa gggcatgaaa ccagcgactg gaacagctac 60
tacgcagaca cgc 73

<210> 28
<211> 83
<212> DNA
<213> Artificial Sequence

<220>
<223> amplicon

<400> 28
gcatggtagc cgaagatttc acagtcaaaa tcggagattt tggtatgacg cgagatatct 60
atgagacaga ctattaccgg aaa 83

<210> 29
<211> 68
<212> DNA
<213> Artificial Sequence

<220>
<223> amplicon

<400> 29
acgcaccggg tgtctgatcc caagttccac cccctccatt caaagataat catcatcaag 60
aaagggca 68

<210> 30
<211> 77
<212> DNA
<213> Artificial Sequence

<220>
<223> amplicon

<400> 30
tgaaccgcag agaccaacag aggaatccag gcacctctac cacgccctcc cagcccaatt 60
ctgcgggtgt ccaagac 77

<210> 31
<211> 79
<212> DNA
<213> Artificial Sequence

<220>
<223> amplicon

<400> 31
gtccccggga tggatgtttt gccaagtcat tgttggataa gcgagatggt agtacaattg 60
tcagacagct tgactgatc 79

<210> 32
<211> 73
<212> DNA
<213> Artificial Sequence

<220>
<223> amplicon

<400> 32
cgaggattgg ttcttcagca agacagagga actgaaccgc gaggtggcca ccaagagtga 60
gctggtgcag agt 73

<210> 33
<211> 80
<212> DNA
<213> Artificial Sequence

<220>
<223> amplicon

<400> 33
actcaagcgg aaattgaagc agataggtct tatcagcaca gtctccgcct cctggattca 60
gtgtctcggc ttcagggagt 80

<210> 34
<211> 71
<212> DNA
<213> Artificial Sequence

<220>
<223> amplicon

<400> 34
cccatccaga acactgattg ctgtcattca agtgaaaggg atggaggtca gaaagggtgc 60
atagaaagca g 71

<210> 35
<211> 79
<212> DNA
<213> Artificial Sequence

<220>
<223> amplicon

<400> 35
   acggatctac cacaccattg catatttgac accccttccc cagccaaata gagctttgag 60
   tttttttgtt ggatatgga 79

<210> 36
<211> 86
<212> DNA
<213> Artificial Sequence

<220>
<223> amplicon

<400> 36
ccatgatgga gaggcagaca tcatgatcaa ctttggccgc tgggagcatg gcgatggata 60
cccctttgac ggtaaggacg gactcc 86

<210> 37
<211> 67
<212> DNA
<213> Artificial Sequence

<220>
<223> amplicon

<400> 37
gagaaccaat ctcaccgaca ggcagctggc agaggaatac ctgtaccgct atggttacac 60
tcgggtg 67

<210> 38
<211> 77
<212> DNA
<213> Artificial Sequence

<220>
<223> amplicon

<400> 38
ccgccctcac ctgaagagaa acgcgctcct tggcggacac tgggggagga gaggaagaag 60
cgcggctaac ttattcc 77

<210> 39
<211> 71
<212> DNA
<213> Artificial Sequence

<220>
<223> amplicon

<400> 39
ggccaggatc tgtggtggta caattgactc tggccttccg agaaggtacc atcaatgtcc 60
acgacgtgga g 71

<210> 40
<211> 77
<212> DNA
<213> Artificial Sequence

<220>
<223> amplicon

<400> 40
cccagctagt gttcctcaga acaaagattg gaaaaagctg gccgagaacc atttatacat 60
agaggaaggg cttacgg 77

<210> 41
<211> 72
<212> DNA
<213> Artificial Sequence

<220>
<223> amplicon

<400> 41
gggagtttcc aagagatgga ctagtgcttg gtcgggtctt ggggtctgga gcgtttggga 60
aggtggttga ag 72

<210> 42
<211> 66
<212> DNA
<213> Artificial Sequence

<220>
<223> amplicon

<400> 42
ccagctctcc ttccagctac agatcaatgt ccctgtccga gtgctggagc taagtgagag 60
ccaccc 66

<210> 43
<211> 74
<212> DNA
<213> Artificial Sequence

<220>
<223> amplicon

<400> 43
agagccagtt gctgtagaac tcaaatctct gctgggcaag gatgttctgt tcttgaagga 60
ctgtgtaggc ccag 74

<210> 44
<211> 69
<212> DNA
<213> Artificial Sequence

<220>
<223> amplicon

<400> 44
agtgggagac acctgacctt tctcaagctg agattgagca gaagatcaag gagtacaatg 60
cccagatca 69

<210> 45
<211> 85
<212> DNA
<213> Artificial Sequence

<220>
<223> amplicon

<400> 45
ccacaagctg aaggcagaca aggcccgcaa gaagctcctg gctgaccagg ctgaggcccg 60
caggtctaag accaaggaag cacgc 85

<210> 46
<211> 75
<212> DNA
<213> Artificial Sequence

<220>
<223> amplicon

<400> 46
ccattctatc atcaacgggt acaaacgagt cctggccttg tctgtggaga cggattacac 60
cttcccactt gctga 75

<210> 47
<211> 66
<212> DNA
<213> Artificial Sequence

<220>
<223> amplicon

<400> 47
gggctactgg cagctacatt gctgggacta atggcaattc caatggcctt gtaccgatgc 60
tgagag 66

<210> 48
<211> 70
<212> DNA
<213> Artificial Sequence

<220>
<223> amplicon

<400> 48
tcacatgcca ctttggtgtt tcataatdtc ctgggagaga ttgaccagca gtatagccgc 60
ttcctgcaag 70

<210> 49
<211> 74

<212> DNA
<213> Artificial Sequence

<220>
<223> amplicon

<400> 49
ccagtggtgg tgatcgttca tggcagccag gacaacaatg cgacggccac tgttctctgg 60
gacaatgctt ttgc 74

<210> 50
<211> 90
<212> DNA
<213> Artificial Sequence

<220>
<223> amplicon

<400> 50
cctggaggct gcaacatacc tcaatcctgt cccaggccgg atcctcctga agccctttc 60
gcagcactgc tatcctccaa agccattgta 90

<210> 51
<211> 70
<212> DNA
<213> Artificial Sequence

<220>
<223> amplicon

<400> 51
tggccctcaa cctcatcttg atggcagcct ctggtgctgt gtgcgaagtg aaggacgttt 60
gtgttggaag 70

<210> 52
<211> 73
<212> DNA
<213> Artificial Sequence

<220>
<223> amplicon

<400> 52
gatggagcag gtggctcagt tcctgaaggc ggctgaggac tctggggtca tcaagactga 60
catgttccag act 73

<210> 53
<211> 69
<212> DNA
<213> Artificial Sequence

<220>
<223> amplicon

<400> 53
tcaccctctg tgacttcatc gtgccctggg acaccctgag caccacccag aagaagagcc 60
tgaaccaca 69

<210> 54
<211> 70
<212> DNA
<213> Artificial Sequence

<220>
<223> amplicon

<400> 54

cgactccgtt ctcagtgtct gacatcttga gtcccctgga ggaaagctac aagaaagtgg 60
gcatggaggg 70

<210> 55
<211> 83
<212> DNA
<213> Artificial Sequence

<220>
<223> amplicon

<400> 55
cctcagcaag acgttatttg aaattacagt gcctctctct caaggcccca aaccagtaac 60
aatcagtttt gccaatcaca ctt 83

<210> 56
<211> 75
<212> DNA
<213> Artificial Sequence

<220>
<223> amplicon

<400> 56
agaggcatcc atgaacttca cacttgcggg ctgcatcagc acacgctcct atcaacccaa 60
gtactgtgga gtttg 75

<210> 57
<211> 77
<212> DNA
<213> Artificial Sequence

<220>
<223> amplicon

<400> 57
gcagttggaa gacacaggaa agtatcccca aattgcagat ttatcaacgg cttttatctt 60
gaaaatagtg ccacgca 77

<210> 58
<211> 76
<212> DNA
<213> Artificial Sequence

<220>
<223> amplicon

<400> 58
agactgtgga gtttgatgtt gttgaaggag aaaagggtgc ggaggcagca aatgttacag 60
gtcctggtgg tgttcc 76

<210> 59
<211> 20
<212> DNA
<213> Artificial Sequence

<220>
<223> forward primer

<400> 59
tagagagccc ctgacaatcc 20

<210> 60
<211> 20
<212> DNA
<213> Artificial Sequence

<220>
<223> reverse primer

<400> 60
ggttggtctt ggaaagagga                                                    20

<210> 61
<211> 25
<212> DNA
<213> Artificial Sequence

<220>
<223> probe

<400> 61
tggctctagc tcctgatgaa gcctc                                              25

<210> 62
<211> 20
<212> DNA
<213> Artificial Sequence

<220>
<223> forward primer

<400> 62
ggacagcagg aatgtgtttc                                                    20

<210> 63
<211> 20
<212> DNA
<213> Artificial Sequence

<220>
<223> reverse primer

<400> 63
acccactcga tttgtttctg                                                    20

<210> 64
<211> 22
<212> DNA
<213> Artificial Sequence

<220>
<223> probe

<400> 64
cattggctcc ccgtgacctg ta                                                 22

<210> 65
<211> 20
<212> DNA
<213> Artificial Sequence

<220>
<223> forward primer

<400> 65
gggatcgaga catgtaagca                                                    20

<210> 66
<211> 21
<212> DNA
<213> Artificial Sequence

<220>

<223> reverse primer

<400> 66
tggaattcat ccaatccaaa t                                                21

<210> 67
<211> 24
<212> DNA
<213> Artificial Sequence

<220>
<223> probe

<400> 67
cggcatcttc aaacctccat gatg                                             24

<210> 68
<211> 20
<212> DNA
<213> Artificial Sequence

<220>
<223> forward primer

<400> 68
ccattcccac cattctacct                                                  20

<210> 69
<211> 20
<212> DNA
<213> Artificial Sequence

<220>
<223> reverse primer

<400> 69
gggaacatag acccaccaat                                                  20

<210> 70
<211> 21
<212> DNA
<213> Artificial Sequence

<220>
<223> probe

<400> 70
acaccccaga cgtcctggcc t                                                21

<210> 71
<211> 18
<212> DNA
<213> Artificial Sequence

<220>
<223> forward primer

<400> 71
agggagatgc cgcttcgt                                                    18

<210> 72
<211> 23
<212> DNA
<213> Artificial Sequence

<220>
<223> reverse primer

<400> 72
ctctcacacc ttgctccaat gta                                              23

<210> 73
<211> 25
<212> DNA
<213> Artificial Sequence

<220>
<223> probe

<400> 73
ccttcatcac agacacagga gggcg                                            25

<210> 74
<211> 27
<212> DNA
<213> Artificial Sequence

<220>
<223> forward primer

<400> 74
cctctgtgct acagattata cctttgc                                          27

<210> 75
<211> 18
<212> DNA
<213> Artificial Sequence

<220>
<223> reverse primer

<400> 75
cactgcagcc ccaatgct                                                    18

<210> 76
<211> 22
<212> DNA
<213> Artificial Sequence

<220>
<223> probe

<400> 76
tacccgccat ccatgatcgc ca                                               22

<210> 77
<211> 20
<212> DNA
<213> Artificial Sequence

<220>
<223> forward primer

<400> 77
tcttgctggc tacgcctctt                                                  20

<210> 78
<211> 21
<212> DNA
<213> Artificial Sequence

<220>
<223> reverse primer

<400> 78
ctgcattgtg gcacagttct g                                                21

<210> 79
<211> 28
<212> DNA
<213> Artificial Sequence

<220>
<223> probe

<400> 79
tgtccctgtt agacgtcctc cgtccata                                         28

<210> 80
<211> 20
<212> DNA
<213> Artificial Sequence

<220>
<223> forward primer

<400> 80
aaagcctcag tcagccaagt                                                  20

<210> 81
<211> 20
<212> DNA
<213> Artificial Sequence

<220>
<223> reverse primer

<400> 81
aaccaaactg tccacagcag                                                  20

<210> 82
<211> 24
<212> DNA
<213> Artificial Sequence

<220>
<223> probe

<400> 82
tcctgagcat tttcaagtcc gcct                                             24

<210> 83
<211> 19
<212> DNA
<213> Artificial Sequence

<220>
<223> forward primer

<400> 83
ggctgctttg ctgcaactg                                                   19

<210> 84
<211> 19
<212> DNA
<213> Artificial Sequence

<220>
<223> reverse primer

<400> 84

cagagcgggc agcagaata                                                    19

<210> 85
<211> 24
<212> DNA
<213> Artificial Sequence

<220>
<223> probe

<400> 85
cgcacagaca agccttactc cgcc                                               24

<210> 86
<211> 20
<212> DNA
<213> Artificial Sequence

<220>
<223> forward primer

<400> 86
gcaagttcca acctggaaag                                                    20

<210> 87
<211> 20
<212> DNA
<213> Artificial Sequence

<220>
<223> reverse primer

<400> 87
catcgcttcc tcgtcataga                                                    20

<210> 88
<211> 24
<212> DNA
<213> Artificial Sequence

<220>
<223> probe

<400> 88
tggctacatc cttgacaaag ccga                                               24

<210> 89
<211> 20
<212> DNA
<213> Artificial Sequence

<220>
<223> forward primer

<400> 89
ctcctcttgc ctacgagtcc                                                    20

<210> 90
<211> 19
<212> DNA
<213> Artificial Sequence

<220>
<223> reverse primer

<400> 90
ctcacctcac cccacgata                                                     19

<210> 91
<211> 24
<212> DNA
<213> Artificial Sequence

<220>
<223> probe

<400> 91
cctctcctcg taggcctctc ggat                                              24

<210> 92
<211> 27
<212> DNA
<213> Artificial Sequence

<220>
<223> forward primer

<400> 92
ttgctataac taagtgcttc tccaaga                                           27

<210> 93
<211> 22
<212> DNA
<213> Artificial Sequence

<220>
<223> reverse primer

<400> 93
gtggaatggc agctcactgt ag                                                22

<210> 94
<211> 22
<212> DNA
<213> Artificial Sequence

<220>
<223> probe

<400> 94
cccaactgag tccccagcac ct                                                22

<210> 95
<211> 19
<212> DNA
<213> Artificial Sequence

<220>
<223> forward primer

<400> 95
aggacgcaag gagggtttg                                                    19

<210> 96
<211> 21
<212> DNA
<213> Artificial Sequence

<220>
<223> reverse primer

<400> 96
gatgtccgcc gagtccttac t                                                 21

<210> 97
<211> 29
<212> DNA
<213> Artificial Sequence

<220>
<223> probe

<400> 97
cagtgcctac agtctcgagt ctgccagtg                                          29

<210> 98
<211> 20
<212> DNA
<213> Artificial Sequence

<220>
<223> forward primer

<400> 98
actccctcta cccttgagca                                                    20

<210> 99
<211> 20
<212> DNA
<213> Artificial Sequence

<220>
<223> reverse primer

<400> 99
caggcctcag ttccttcagt                                                    20

<210> 100
<211> 24
<212> DNA
<213> Artificial Sequence

<220>
<223> probe

<400> 100
cagaagaaca gctcagggac ccct                                               24

<210> 101
<211> 20
<212> DNA
<213> Artificial Sequence

<220>
<223> forward primer

<400> 101
tgtcgatgga cttccagaac                                                    20

<210> 102
<211> 19
<212> DNA
<213> Artificial Sequence

<220>
<223> reverse primer

<400> 102
attgggacag cttggatca                                                     19

<210> 103

<211> 18
<212> DNA
<213> Artificial Sequence

<220>
<223> probe

<400> 103
cacctgggca gctgccaa                                                    18

<210> 104
<211> 25
<212> DNA
<213> Artificial Sequence

<220>
<223> forward primer

<400> 104
gctagtactt tgatgctccc ttgat                                            25

<210> 105
<211> 20
<212> DNA
<213> Artificial Sequence

<220>
<223> reverse primer

<400> 105
gaacagctgg aggccaagtc                                                  20

<210> 106
<211> 22
<212> DNA
<213> Artificial Sequence

<220>
<223> probe

<400> 106
ccagagagcc tccctgcagc ca                                               22

<210> 107
<211> 20
<212> DNA
<213> Artificial Sequence

<220>
<223> forward primer

<400> 107
accgtaggct ctgctctgaa                                                  20

<210> 108
<211> 20
<212> DNA
<213> Artificial Sequence

<220>
<223> reverse primer

<400> 108
tggtccaggt ggaaaacttc                                                  20

<210> 109
<211> 20

<212> DNA
<213> Artificial Sequence

<220>
<223> probe

<400> 109
aggcagccag acccacagga                                                    20

<210> 110
<211> 20
<212> DNA
<213> Artificial Sequence

<220>
<223> forward primer

<400> 110
ggctcccgaa tctatctttg                                                    20

<210> 111
<211> 20
<212> DNA
<213> Artificial Sequence

<220>
<223> reverse primer

<400> 111
tcccacagca atactccgta                                                    20

<210> 112
<211> 24
<212> DNA
<213> Artificial Sequence

<220>
<223> probe

<400> 112
ctacagcacc aagagcgacg tgtg                                               24

<210> 113
<211> 19
<212> DNA
<213> Artificial Sequence

<220>
<223> forward primer

<400> 113
gaacgccaag caccacaag                                                     19

<210> 114
<211> 20
<212> DNA
<213> Artificial Sequence

<220>
<223> reverse primer

<400> 114
ccagggtcga cactgctcat                                                    20

<210> 115
<211> 23
<212> DNA

<213> Artificial Sequence

<220>
<223> probe

<400> 115
aagccaggcc ccgaggacaa gtt                                              23

<210> 116
<211> 24
<212> DNA
<213> Artificial Sequence

<220>
<223> forward primer

<400> 116
aagtcctcga tacgcactat agca                                             24

<210> 117
<211> 19
<212> DNA
<213> Artificial Sequence

<220>
<223> reverse primer

<400> 117
ctggcatgtg gcacatgag                                                   19

<210> 118
<211> 21
<212> DNA
<213> Artificial Sequence

<220>
<223> probe

<400> 118
cccggatggt ctccacgtca t                                                21

<210> 119
<211> 26
<212> DNA
<213> Artificial Sequence

<220>
<223> forward primer

<400> 119
ggataattca gacaacaaca ccatct                                           26

<210> 120
<211> 25
<212> DNA
<213> Artificial Sequence

<220>
<223> reverse primer

<400> 120
tgaagtaatc agccacagac tcaat                                            25

<210> 121
<211> 29
<212> DNA
<213> Artificial Sequence

<220>
<223> probe

<400> 121
tcaattgtaa cattctcacc caggccttg                                          29

<210> 122
<211> 24
<212> DNA
<213> Artificial Sequence

<220>
<223> forward primer

<400> 122
cacacagatc tcctactcca tcca                                               24

<210> 123
<211> 21
<212> DNA
<213> Artificial Sequence

<220>
<223> reverse primer

<400> 123
ggtccagcag tgtctcctga a                                                  21

<210> 124
<211> 26
<212> DNA
<213> Artificial Sequence

<220>
<223> probe

<400> 124
catgctgcat cctaaggctc ctcagg                                             26

<210> 125
<211> 20
<212> DNA
<213> Artificial Sequence

<220>
<223> forward primer

<400> 125
gagaccctgc tgtcccagaa                                                    20

<210> 126
<211> 23
<212> DNA
<213> Artificial Sequence

<220>
<223> reverse primer

<400> 126
ggttgtagtc agcgaaggag atc                                                23

<210> 127
<211> 26
<212> DNA
<213> Artificial Sequence

<220>
<223> probe

<400> 127
   tcccacaatg aaggtcttgc ctccct 26

<210> 128
<211> 23
<212> DNA
<213> Artificial Sequence

<220>
<223> forward primer

<400> 128
tgacaccctt accttcctga gaa 23

<210> 129
<211> 29
<212> DNA
<213> Artificial Sequence

<220>
<223> reverse primer

<400> 129
aaaaacacga gtcaaaaata gaagtcact 29

<210> 130
<211> 21
<212> DNA
<213> Artificial Sequence

<220>
<223> probe

<400> 130
tctgctttcc gcgctcccag g 21

<210> 131
<211> 20
<212> DNA
<213> Artificial Sequence

<220>
<223> forward primer

<400> 131
aggctgctgg aggtcatctc 20

<210> 132
<211> 19
<212> DNA
<213> Artificial Sequence

<220>
<223> reverse primer

<400> 132
cttcctgcgg ccacagtct 19

<210> 133
<211> 21
<212> DNA
<213> Artificial Sequence

<220>

<223> probe

<400> 133
ccagaggccg tttcttggcc g                                                21

<210> 134
<211> 20
<212> DNA
<213> Artificial Sequence

<220>
<223> forward primer

<400> 134
cggtgtgaga agtgcagcaa                                                  20

<210> 135
<211> 19
<212> DNA
<213> Artificial Sequence

<220>
<223> reverse primer

<400> 135
cctctcgcaa gtgctccat                                                   19

<210> 136
<211> 24
<212> DNA
<213> Artificial Sequence

<220>
<223> probe

<400> 136
ccagaccata gcacactcgg gcac                                             24

<210> 137
<211> 24
<212> DNA
<213> Artificial Sequence

<220>
<223> forward primer

<400> 137
tccaggatgt taggaactgt gaag                                             24

<210> 138
<211> 22
<212> DNA
<213> Artificial Sequence

<220>
<223> reverse primer

<400> 138
gcgtgtctgc gtagtagctg tt                                               22

<210> 139
<211> 24
<212> DNA
<213> Artificial Sequence

<220>
<223> probe

<400> 139
agtcgctggt ttcatgccct tcca                                              24

<210> 140
<211> 21
<212> DNA
<213> Artificial Sequence

<220>
<223> forward primer

<400> 140
gcatggtagc cgaagatttc a                                                 21

<210> 141
<211> 30
<212> DNA
<213> Artificial Sequence

<220>
<223> reverse primer

<400> 141
tttccggtaa tagtctgtct catagatatc                                        30

<210> 142
<211> 28
<212> DNA
<213> Artificial Sequence

<220>
<223> probe

<400> 142
cgcgtcatac caaaatctcc gattttga                                          28

<210> 143
<211> 17
<212> DNA
<213> Artificial Sequence

<220>
<223> forward primer

<400> 143
acgcaccggg tgtctga                                                      17

<210> 144
<211> 24
<212> DNA
<213> Artificial Sequence

<220>
<223> reverse primer

<400> 144
tgccctttct tgatgatgat tatc                                              24

<210> 145
<211> 24
<212> DNA
<213> Artificial Sequence

<220>
<223> probe

<400> 145
cccaagttcc accccctcca ttca                                              24

<210> 146
<211> 20
<212> DNA
<213> Artificial Sequence

<220>
<223> forward primer

<400> 146
tgaaccgcag agaccaacag                                                   20

<210> 147
<211> 20
<212> DNA
<213> Artificial Sequence

<220>
<223> reverse primer

<400> 147
gtcttggaca cccgcagaat                                                   20

<210> 148
<211> 25
<212> DNA
<213> Artificial Sequence

<220>
<223> probe

<400> 148
atccaggcac ctctaccacg ccctc                                             25

<210> 149
<211> 18
<212> DNA
<213> Artificial Sequence

<220>
<223> forward primer

<400> 149
gtccccggga tggatgtt                                                     18

<210> 150
<211> 25
<212> DNA
<213> Artificial Sequence

<220>
<223> reverse primer

<400> 150
gatcagtcaa gctgtctgac aattg                                             25

<210> 151
<211> 29
<212> DNA
<213> Artificial Sequence

<220>
<223> probe

<400> 151
   catctcgctt atccaacaat gacttggca                                         29

<210> 152
<211> 21
<212> DNA
<213> Artificial Sequence

<220>
<223> forward primer

<400> 152
cgaggattgg ttcttcagca a 21

<210> 153
<211> 22
<212> DNA
<213> Artificial Sequence

<220>
<223> reverse primer

<400> 153
actctgcacc agctcactgt tg 22

<210> 154
<211> 24
<212> DNA
<213> Artificial Sequence

<220>
<223> probe

<400> 154
cacctcgcgg ttcagttcct ctgt 24

<210> 155
<211> 21
<212> DNA
<213> Artificial Sequence

<220>
<223> forward primer

<400> 155
actcaagcgg aaattgaagc a 21

<210> 156
<211> 21
<212> DNA
<213> Artificial Sequence

<220>
<223> reverse primer

<400> 156
actccctgaa gccgagacac t 21

<210> 157
<211> 28
<212> DNA
<213> Artificial Sequence

<220>
<223> probe

<400> 157
aggtcttatc agcacagtct ccgcctcc 28

<210> 158
<211> 20
<212> DNA
<213> Artificial Sequence

<220>
<223> forward primer

<400> 158
cccatccaga acactgattg                                                    20

<210> 159
<211> 21
<212> DNA
<213> Artificial Sequence

<220>
<223> reverse primer

<400> 159
ctgctttcta tgcacccttt c                                                  21

<210> 160
<211> 26
<212> DNA
<213> Artificial Sequence

<220>
<223> probe

<400> 160
tgacctccat ccctttcact tgaatg                                             26

<210> 161
<211> 21
<212> DNA
<213> Artificial Sequence

<220>
<223> forward primer

<400> 161
acggatctac cacaccattg c                                                  21

<210> 162
<211> 26
<212> DNA
<213> Artificial Sequence

<220>
<223> reverse primer

<400> 162
tccatatcca acaaaaaaac tcaaag                                             26

<210> 163
<211> 22
<212> DNA
<213> Artificial Sequence

<220>
<223> probe

<400> 163
tttgacaccc cttccccagc ca                                                 22

<210> 164
<211> 20
<212> DNA
<213> Artificial Sequence

<220>
<223> forward primer

<400> 164
ccatgatgga gaggcagaca                                                    20

<210> 165
<211> 21
<212> DNA
<213> Artificial Sequence

<220>
<223> reverse primer

<400> 165
ggagtccgtc cttaccgtca a                                                  21

<210> 166
<211> 24
<212> DNA
<213> Artificial Sequence

<220>
<223> probe

<400> 166
ctgggagcat ggcgatggat accc                                               24

<210> 167
<211> 20
<212> DNA
<213> Artificial Sequence

<220>
<223> forward primer

<400> 167
gagaaccaat ctcaccgaca                                                    20

<210> 168
<211> 20
<212> DNA
<213> Artificial Sequence

<220>
<223> reverse primer

<400> 168
cacccgagtg taaccatagc                                                    20

<210> 169
<211> 24
<212> DNA
<213> Artificial Sequence

<220>
<223> probe

<400> 169
acaggtattc ctctgccagc tgcc                                               24

<210> 170

<211> 19
<212> DNA
<213> Artificial Sequence

<220>
<223> forward primer

<400> 170
ccgccctcac ctgaagaga                                                    19

<210> 171
<211> 22
<212> DNA
<213> Artificial Sequence

<220>
<223> reverse primer

<400> 171
ggaataagtt agccgcgctt ct                                                22

<210> 172
<211> 21
<212> DNA
<213> Artificial Sequence

<220>
<223> probe

<400> 172
cccagtgtcc gccaaggagc g                                                 21

<210> 173
<211> 20
<212> DNA
<213> Artificial Sequence

<220>
<223> forward primer

<400> 173
ggccaggatc tgtggtggta                                                   20

<210> 174
<211> 20
<212> DNA
<213> Artificial Sequence

<220>
<223> reverse primer

<400> 174
ctccacgtcg tggacattga                                                   20

<210> 175
<211> 23
<212> DNA
<213> Artificial Sequence

<220>
<223> probe

<400> 175
ctctggcctt ccgagaaggt acc                                               23

<210> 176
<211> 20

<212> DNA
<213> Artificial Sequence

<220>
<223> forward primer

<400> 176
cccagctagt gttcctcaga 20

<210> 177
<211> 20
<212> DNA
<213> Artificial Sequence

<220>
<223> reverse primer

<400> 177
ccgtaagccc ttcctctatg 20

<210> 178
<211> 24
<212> DNA
<213> Artificial Sequence

<220>
<223> probe

<400> 178
tcggccagct ttttccaatc tttg 24

<210> 179
<211> 20
<212> DNA
<213> Artificial Sequence

<220>
<223> forward primer

<400> 179
gggagtttcc aagagatgga 20

<210> 180
<211> 20
<212> DNA
<213> Artificial Sequence

<220>
<223> reverse primer

<400> 180
cttcaaccac cttcccaaac 20

<210> 181
<211> 23
<212> DNA
<213> Artificial Sequence

<220>
<223> probe

<400> 181
cccaagaccc gaccaagcac tag 23

<210> 182
<211> 20
<212> DNA

<213> Artificial Sequence

<220>
<223> forward primer

<400> 182
ccagctctcc ttccagctac                                                    20

<210> 183
<211> 20
<212> DNA
<213> Artificial Sequence

<220>
<223> reverse primer

<400> 183
gggtggctct cacttagctc                                                    20

<210> 184
<211> 24
<212> DNA
<213> Artificial Sequence

<220>
<223> probe

<400> 184
atcaatgtcc ctgtccgagt gctg                                               24

<210> 185
<211> 24
<212> DNA
<213> Artificial Sequence

<220>
<223> forward primer

<400> 185
agagccagtt gctgtagaac tcaa                                               24

<210> 186
<211> 21
<212> DNA
<213> Artificial Sequence

<220>
<223> reverse primer

<400> 186
ctgggcctac acagtccttc a                                                  21

<210> 187
<211> 27
<212> DNA
<213> Artificial Sequence

<220>
<223> probe

<400> 187
tctctgctgg gcaaggatgt tctgttc                                            27

<210> 188
<211> 20
<212> DNA
<213> Artificial Sequence

<220>
<223> forward primer

<400> 188
agtgggagac acctgacctt                                                    20

<210> 189
<211> 20
<212> DNA
<213> Artificial Sequence

<220>
<223> reverse primer

<400> 189
tgatctgggc attgtactcc                                                    20

<210> 190
<211> 29
<212> DNA
<213> Artificial Sequence

<220>
<223> probe

<400> 190
ttgatcttct gctcaatctc agcttgaga                                          29

<210> 191
<211> 20
<212> DNA
<213> Artificial Sequence

<220>
<223> forward primer

<400> 191
ccacaagctg aaggcagaca                                                    20

<210> 192
<211> 21
<212> DNA
<213> Artificial Sequence

<220>
<223> reverse primer

<400> 192
gcgtgcttcc ttggtcttag a                                                  21

<210> 193
<211> 22
<212> DNA
<213> Artificial Sequence

<220>
<223> probe

<400> 193
cgcaagaagc tcctggctga cc                                                 22

<210> 194
<211> 24
<212> DNA
<213> Artificial Sequence

<220>
<223> forward primer

<400> 194
ccattctatc atcaacgggt acaa                                              24

<210> 195
<211> 23
<212> DNA
<213> Artificial Sequence

<220>
<223> reverse primer

<400> 195
tcagcaagtg ggaaggtgta atc                                               23

<210> 196
<211> 25
<212> DNA
<213> Artificial Sequence

<220>
<223> probe

<400> 196
tctccacaga caaggccagg actcg                                             25

<210> 197
<211> 20
<212> DNA
<213> Artificial Sequence

<220>
<223> forward primer

<400> 197
gggctactgg cagctacatt                                                   20

<210> 198
<211> 20
<212> DNA
<213> Artificial Sequence

<220>
<223> reverse primer

<400> 198
ctctcagcat cggtacaagg                                                   20

<210> 199
<211> 25
<212> DNA
<213> Artificial Sequence

<220>
<223> probe

<400> 199
cattggaatt gccattagtc ccagc                                             25

<210> 200
<211> 20
<212> DNA
<213> Artificial Sequence

<220>

<223> forward primer

<400> 200
tcacatgcca ctttggtgtt                                                    20

<210> 201
<211> 20
<212> DNA
<213> Artificial Sequence

<220>
<223> reverse primer

<400> 201
cttgcaggaa gcggctatac                                                    20

<210> 202
<211> 22
<212> DNA
<213> Artificial Sequence

<220>
<223> probe

<400> 202
tcctgggaga gattgaccag ca                                                 22

<210> 203
<211> 20
<212> DNA
<213> Artificial Sequence

<220>
<223> forward primer

<400> 203
ccagtggtgg tgatcgttca                                                    20

<210> 204
<211> 21
<212> DNA
<213> Artificial Sequence

<220>
<223> reverse primer

<400> 204
gcaaaagcat tgtcccagag a                                                  21

<210> 205
<211> 23
<212> DNA
<213> Artificial Sequence

<220>
<223> probe

<400> 205
cagccaggac aacaatgcga cgg                                                23

<210> 206
<211> 20
<212> DNA
<213> Artificial Sequence

<220>
<223> forward primer

<400> 206
cctggaggct gcaacatacc                                                    20

<210> 207
<211> 23
<212> DNA
<213> Artificial Sequence

<220>
<223> reverse primer

<400> 207
tagaatggct ttggaggata gca                                                23

<210> 208
<211> 25
<212> DNA
<213> Artificial Sequence

<220>
<223> probe

<400> 208
atcctcctga agcccttttc gcagc                                              25

<210> 209
<211> 20
<212> DNA
<213> Artificial Sequence

<220>
<223> forward primer

<400> 209
tggccctcaa cctcatcttg                                                    20

<210> 210
<211> 22
<212> DNA
<213> Artificial Sequence

<220>
<223> reverse primer

<400> 210
cttccaacac aaacgtcctt ca                                                 22

<210> 211
<211> 23
<212> DNA
<213> Artificial Sequence

<220>
<223> probe

<400> 211
ttcgcacaca gcaccagagg ctg                                                23

<210> 212
<211> 20
<212> DNA
<213> Artificial Sequence

<220>
<223> forward primer

<400> 212
gatggagcag gtggctcagt                                                    20

<210> 213
<211> 25
<212> DNA
<213> Artificial Sequence

<220>
<223> reverse primer

<400> 213
agtctggaac atgtcagtct tgatg                                              25

<210> 214
<211> 24
<212> DNA
<213> Artificial Sequence

<220>
<223> probe

<400> 214
cccagagtcc tcagccgcct tcag                                               24

<210> 215
<211> 22
<212> DNA
<213> Artificial Sequence

<220>
<223> forward primer

<400> 215
tcaccctctg tgacttcatc gt                                                 22

<210> 216
<211> 22
<212> DNA
<213> Artificial Sequence

<220>
<223> reverse primer

<400> 216
tgtggttcag gctcttcttc tg                                                 22

<210> 217
<211> 22
<212> DNA
<213> Artificial Sequence

<220>
<223> probe

<400> 217
ccctgggaca ccctgagcac ca                                                 22

<210> 218
<211> 22
<212> DNA
<213> Artificial Sequence

<220>
<223> forward primer

<400> 218

cgactccgtt ctcagtgtct ga                                                22

<210> 219
<211> 19
<212> DNA
<213> Artificial Sequence

<220>
<223> reverse primer

<400> 219
ccctccatgc ccactttct                                                   19

<210> 220
<211> 24
<212> DNA
<213> Artificial Sequence

<220>
<223> probe

<400> 220
atcttgagtc ccctggagga aagc                                             24

<210> 221
<211> 25
<212> DNA
<213> Artificial Sequence

<220>
<223> forward primer

<400> 221
cctcagcaag acgttatttg aaatt                                            25

<210> 222
<211> 24
<212> DNA
<213> Artificial Sequence

<220>
<223> reverse primer

<400> 222
aagtgtgatt ggcaaaactg attg                                             24

<210> 223
<211> 26
<212> DNA
<213> Artificial Sequence

<220>
<223> probe

<400> 223
cctctctctc aaggccccaa accagt                                           26

<210> 224
<211> 22
<212> DNA
<213> Artificial Sequence

<220>
<223> forward primer

<400> 224
agaggcatcc atgaacttca ca                                               22

<210> 225
<211> 24
<212> DNA
<213> Artificial Sequence

<220>
<223> reverse primer

<400> 225
caaactccac agtacttggg ttga                                              24

<210> 226
<211> 20
<212> DNA
<213> Artificial Sequence

<220>
<223> probe

<400> 226
cgggctgcat cagcacacgc                                                   20

<210> 227
<211> 23
<212> DNA
<213> Artificial Sequence

<220>
<223> forward primer

<400> 227
gcagttggaa gacacaggaa agt                                               23

<210> 228
<211> 21
<212> DNA
<213> Artificial Sequence

<220>
<223> reverse primer

<400> 228
tgcgtggcac tattttcaag a                                                 21

<210> 229
<211> 27
<212> DNA
<213> Artificial Sequence

<220>
<223> probe

<400> 229
tccccaaatt gcagatttat caacggc                                           27

<210> 230
<211> 25
<212> DNA
<213> Artificial Sequence

<220>
<223> forward primer

<400> 230
agactgtgga gtttgatgtt gttga                                             25

<210> 231
<211> 22
<212> DNA
<213> Artificial Sequence

<220>
<223> reverse primer

<400> 231
ggaacaccac caggacctgt aa                                                22

<210> 232
<211> 23
<212> DNA
<213> Artificial Sequence

<220>
<223> probe

<400> 232
ttgctgcctc cgcacccttt tct                                               23